(19)

**Europäisches Patentamt**
**European Patent Office**
**Office européen des brevets**

(11) **EP 4 255 318 B1**

(12) **EUROPEAN PATENT SPECIFICATION**

(45) Date of publication and mention
of the grant of the patent:
**05.11.2025 Bulletin 2025/45**

(21) Application number: **22800817.3**

(22) Date of filing: **25.10.2022**

(51) International Patent Classification (IPC):
***A61B 17/068*** (2006.01)

(52) Cooperative Patent Classification (CPC):
**A61B 17/07207;** A61B 2017/00017;
A61B 2017/00115; A61B 2017/00154;
A61B 2017/00353; A61B 2017/00398;
A61B 2017/00734; A61B 2017/07285

(86) International application number:
**PCT/IB2022/060230**

(87) International publication number:
**WO 2023/073545 (04.05.2023 Gazette 2023/18)**

(54) **ALTERNATE MEANS TO ESTABLISH RESISTIVE LOAD FORCE**

ALTERNATIVE MITTEL ZUR HERSTELLUNG EINER WIDERSTANDSLASTKRAFT

MOYENS ALTERNATIFS POUR ÉTABLIR UNE FORCE DE CHARGE RÉSISTIVE

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **28.10.2021 US 202117513705**

(43) Date of publication of application:
**11.10.2023 Bulletin 2023/41**

(73) Proprietor: **Cilag GmbH International**
**6300 Zug (CH)**

(72) Inventor: **LEIMBACH, Richard L.**
**Cincinnati, Ohio 45242 (US)**

(74) Representative: **Carpmaels & Ransford LLP**
**One Southampton Row**
**London WC1B 5HA (GB)**

(56) References cited:
**US-A1- 2009 090 763    US-A1- 2019 200 977**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## BACKGROUND

The present disclosure relates to surgical instruments and surgical robots, including robotic tool attachments for use with a surgical robot.

## FIGURES

The various aspects described herein, both as to organization and methods of operation, together with further objects and advantages thereof, may best be understood by reference to the following description, taken in conjunction with the accompanying drawings as follows.

FIG. 1 is a perspective view of a surgical instrument that has a shaft assembly and an end effector in accordance with one or more aspects of the present disclosure.

FIG. 2 is an exploded assembly view of a portion of the surgical instrument of FIG. 1 according to one aspect of this disclosure.

FIG. 3 is an exploded view of an end effector of the surgical instrument of FIG. 1 according to one aspect of this disclosure.

FIG. 4 illustrates a logic diagram of a control system of a surgical instrument or tool, in accordance with at least one aspect of the present disclosure.

FIG. 5 illustrates a control circuit configured to control aspects of the surgical instrument or tool, in accordance with at least one aspect of the present disclosure.

FIG. 6 illustrates a combinational logic circuit configured to control aspects of the surgical instrument or tool, in accordance with at least one aspect of the present disclosure.

FIG. 7 illustrates a sequential logic circuit configured to control aspects of the surgical instrument or tool, in accordance with at least one aspect of the present disclosure.

FIG. 8 illustrates a surgical instrument or tool comprising a plurality of motors which can be activated to perform various functions, in accordance with at least one aspect of the present disclosure.

FIG. 9 is a schematic diagram of a robotic surgical instrument configured to operate a surgical tool described herein, in accordance with at least one aspect of the present disclosure.

FIG. 10 illustrates a block diagram of a surgical instrument programmed to control the distal translation of a displacement member, in accordance with at least one aspect of the present disclosure.

FIG. 11 is a schematic diagram of a surgical instrument configured to control various functions, in accordance with at least one aspect of the present disclosure.

FIG. 12 is a logic flow diagram of a process depicting a control program or a logic configuration for determining a resistive load force based on battery voltage, velocity, and Pulse Width Modulation (PWM) according to one aspect of this disclosure.

FIG. 13 is a graphical illustration of a resistive load force versus velocity curve at various motor input voltages characterizing various firings of a surgical instrument according to one aspect of this disclosure.

FIG. 14 is a logic flow diagram of a process depicting a control program or a logic configuration for determining a resistive load force based on based on a baseline battery voltage, an activated battery voltage, and actuator velocity according to one aspect of this disclosure.

FIG. 15 is a graphical illustration of a battery power output, battery voltage decrease, actuator velocity, and resistive load force versus time cure characterizing the firing of a surgical instrument according to one aspect of this disclosure.

Corresponding reference characters indicate corresponding parts throughout the several views. The exemplifications set out herein illustrate various disclosed aspects, in one form, and such exemplifications are not to be construed as limiting the scope thereof in any manner.

US 2009/090763 A1 discloses a powered surgical stapler. The stapler includes a housing, an endoscopic portion extending distally from the housing and defining a first longitudinal axis, a drive motor disposed at least partially within a housing and a firing rod disposed in mechanical cooperation with the drive motor. The firing rod is rotatable by the motor about the first longitudinal axis extending therethrough. The stapler also includes an end effector disposed adjacent a distal portion of the endoscopic portion. The end effector is in mechanical cooperation with the firing rod so that the firing rod drives a surgical function of the end effector. The stapler further includes a control system having a plurality of sensors coupled to the drive motor, the firing rod, the loading unit and the end effector, the plurality of sensors configured to detect operating parameters thereof. The control system also includes a microcontroller coupled to the plurality of sensors and being configured to determine operating status of the powered surgical stapler as a function of the detected operating parameters.

US 2019/200977 A1 discloses a method for controlling a surgical instrument. In at least one instance, the surgical instrument comprises a shroud and the operation of the surgical instrument is modified based on input from a sensing circuit configured to sense a parameter of the shroud. In certain instances, the surgical instrument comprises a strain gage circuit and the operation of the surgical instrument is modified based on input from the strain gage circuit.

## DESCRIPTION

The present disclosure relates to surgical instruments and, in various arrangements, to surgical stapling and cutting instruments that are designed to staple and cut tissue. As surgical instruments are used to staple and cut tissue, aspects of the instruments' operation may be monitored, analyzed, or adjusted based on various operating parameters, such as, for example, a resistive load force encountered when firing the instrument. Accordingly, it may be useful to measure, calculate, or otherwise determine various operating parameters of a surgical instrument. Moreover, it may be useful to store data related to these operating parameters in a storage medium associated with the surgical instrument.

Before explaining various aspects of surgical systems, instruments, and staple cartridges in detail, it should be noted that the illustrative examples are not limited in application or use to the details of construction and arrangement of parts illustrated in the accompanying drawings and description. The illustrative examples may be implemented or incorporated in other aspects, variations and modifications, and may be practiced or carried out in various ways. Further, unless otherwise indicated, the terms and expressions employed herein have been chosen for the purpose of describing the illustrative examples for the convenience of the reader and are not for the purpose of limitation thereof. Also, it will be appreciated that one or more of the following-described aspects, expressions of aspects, and/or examples, can be combined with any one or more of the other following-described aspects, expressions of aspects and/or examples.

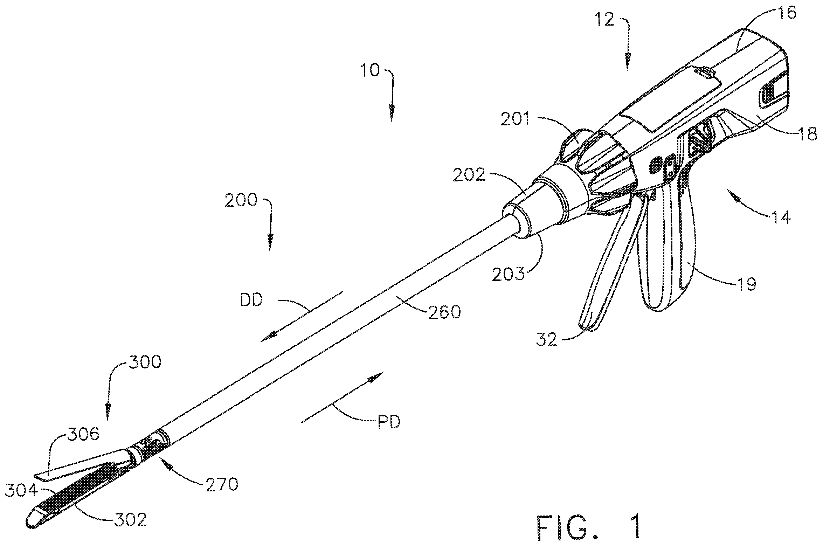

FIGS. 1-3 depict a motor-driven surgical instrument 10 for cutting and fastening that may or may not be reused. In the illustrated examples, the surgical instrument 10 includes a housing 12 that comprises a handle assembly 14 that is configured to be grasped, manipulated, and actuated by the clinician. The housing 12 is configured for operable attachment to an interchangeable shaft assembly 200 that has an end effector 300 operably coupled thereto that is configured to perform one or more surgical tasks or procedures. In accordance with the present disclosure, various forms of interchangeable shaft assemblies may be effectively employed in connection with robotically controlled surgical systems. The term "housing" may encompass a housing or similar portion of a robotic system that houses or otherwise operably supports at least one drive system configured to generate and apply at least one control motion that could be used to actuate interchangeable shaft assemblies. The term "frame" may refer to a portion of a handheld surgical instrument. The term "frame" also may represent a portion of a robotically controlled surgical instrument and/or a portion of the robotic system that may be used to operably control a surgical instrument. Interchangeable shaft assemblies may be employed with various robotic systems, instruments, components, and methods disclosed in U.S. Patent No. 9,072,535, entitled SURGICAL STAPLING INSTRUMENTS WITH ROTATABLE STAPLE DEPLOYMENT ARRANGEMENTS.

FIG. 1 is a perspective view of a surgical instrument 10 that has an interchangeable shaft assembly 200 operably coupled thereto according to one aspect of this disclosure. The housing 12 includes an end effector 300 that comprises a surgical cutting and fastening device configured to operably support a surgical staple cartridge 304 therein. The housing 12 may be configured for use in connection with interchangeable shaft assemblies that include end effectors that are adapted to support different sizes and types of staple cartridges, have different shaft lengths, sizes, and types. The housing 12 may be employed with a variety of interchangeable shaft assemblies, including assemblies configured to apply other motions and forms of energy such as, radio frequency (RF) energy, ultrasonic energy, and/or motion to end effector arrangements adapted for use in connection with various surgical applications and procedures. The end effectors, shaft assemblies, handles, surgical instruments, and/or surgical instrument systems can utilize any suitable fastener, or fasteners, to fasten tissue. For instance, a fastener cartridge comprising a plurality of fasteners removably stored therein can be removably inserted into and/or attached to the end effector of a shaft assembly.

The handle assembly 14 may comprise a pair of interconnectable handle housing segments 16, 18 interconnected by screws, snap features, adhesive, etc. The handle housing segments 16, 18 cooperate to form a pistol grip portion 19 that can be gripped and manipulated by the clinician. The handle assembly 14 operably supports a plurality of drive systems configured to generate and apply control motions to corresponding portions of the interchangeable shaft assembly that is operably attached thereto.

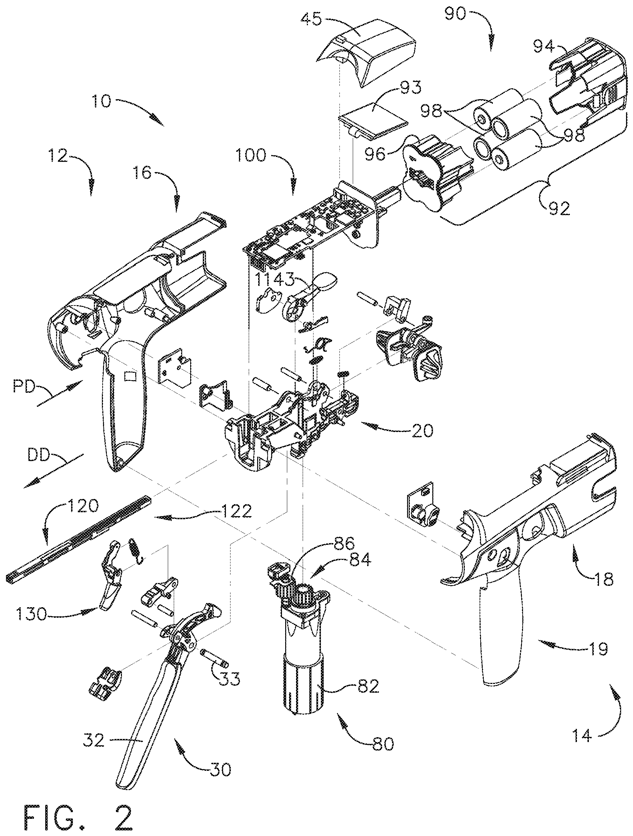

FIG. 2 is an exploded assembly view of a portion of the surgical instrument 10 of FIG. 1 according to one aspect of this disclosure. The handle assembly 14 may include a frame 20 that operably supports a plurality of drive systems. The frame 20 can operably support a "first" or closure drive system 30, which can apply closing and opening motions to the interchangeable shaft assembly 200. The closure drive system 30 may include an actuator such as a closure trigger 32 pivotally supported by the frame 20. The closure trigger 32 is pivotally coupled to the handle assembly 14 by a pivot pin 33 to enable the closure trigger 32 to be manipulated by a clinician. When the clinician grips the pistol grip portion 19 of the handle assembly 14, the closure trigger 32 can pivot from a starting or "unactuated" position to an "actuated" position and more particularly to a fully compressed or fully actuated position.

The handle assembly 14 and the frame 20 may operably support a firing drive system 80 configured to apply firing motions to corresponding portions of the interchangeable shaft assembly attached thereto. The firing drive system 80 may employ an electric motor 82 located in the pistol grip portion 19 of the handle assembly 14. The electric motor 82 may be a DC brushed motor having a maximum rotational speed of approximately 25,000 RPM, for example. In other arrangements, the motor may include a brushless motor, a cordless motor, a synchronous motor, a stepper motor, or any other suitable electric motor. The electric motor 82 may be powered by a power source 90 that may comprise a removable power pack 92. The removable power pack 92 may comprise a proximal housing portion 94 configured to attach to a distal housing portion 96. The proximal housing portion 94 and the distal housing portion 96 are configured to operably support a plurality of batteries 98 therein. Batteries 98 may each comprise, for example, a Lithium Ion (LI) or other suitable battery. The distal housing portion 96 is configured for removable operable attachment to a control circuit board 100, which is operably coupled to the electric motor 82. Several batteries 98 connected in series may power the surgical instrument 10. The power source 90 may be replaceable and/or rechargeable.

The electric motor 82 can include a rotatable shaft (not shown) that operably interfaces with a gear reducer assembly 84 mounted in meshing engagement with a with a set, or rack, of drive teeth 122 on a longitudinally movable drive member 120. The longitudinally movable drive member 120 has a rack of drive teeth 122 formed thereon for meshing engagement with a corresponding drive gear 86 of the gear reducer assembly 84.

In use, a voltage polarity provided by the power source 90 can operate the electric motor 82 in a clockwise direction wherein the voltage polarity applied to the electric motor by the battery can be reversed in order to operate the electric motor 82 in a counter-clockwise direction. When the electric motor 82 is rotated in one direction, the longitudinally movable drive member 120 will be axially driven in the distal direction "DD." When the electric motor 82 is driven in the opposite rotary direction, the longitudinally movable drive member 120 will be axially driven in a proximal direction "PD." The handle assembly 14 can include a switch that can be configured to reverse the polarity applied to the electric motor 82 by the power source 90. The handle assembly 14 may include a sensor configured to detect the position of the longitudinally movable drive member 120 and/or the direction in which the longitudinally movable drive member 120 is being moved.

Actuation of the electric motor 82 can be controlled by a firing trigger 130 that is pivotally supported on the handle assembly 14. The firing trigger 130 may be pivoted between an unactuated position and an actuated position.

Turning back to FIG. 1, the interchangeable shaft assembly 200 includes an end effector 300 comprising an elongated channel 302 configured to operably support a surgical staple cartridge 304 therein. The end effector 300 may include an anvil 306 that is pivotally supported relative to the elongated channel 302. The interchangeable shaft assembly 200 may include an articulation joint 270. Construction and operation of the end effector 300 and the articulation joint 270 are set forth in U.S. Patent Application Publication No. 2014/0263541, entitled ARTICULATABLE SURGICAL INSTRUMENT COMPRISING AN ARTICULATION LOCK. The interchangeable shaft assembly 200 may include a proximal housing or nozzle 201 comprised of nozzle portions 202, 203. The interchangeable shaft assembly 200 may include a closure tube 260 extending along a shaft axis SA that can be utilized to close and/or open the anvil 306 of the end effector 300.

Again referring to FIG. 1, the closure tube 260 is translated distally (direction "DD") to close the anvil 306, for example, in response to the actuation of the closure trigger 32 in the manner described in the aforementioned reference U.S. Patent Application Publication No. 2014/0263541. The anvil 306 is opened by proximally translating the closure tube 260. In the anvil-open position, the closure tube 260 is moved to its proximal position.

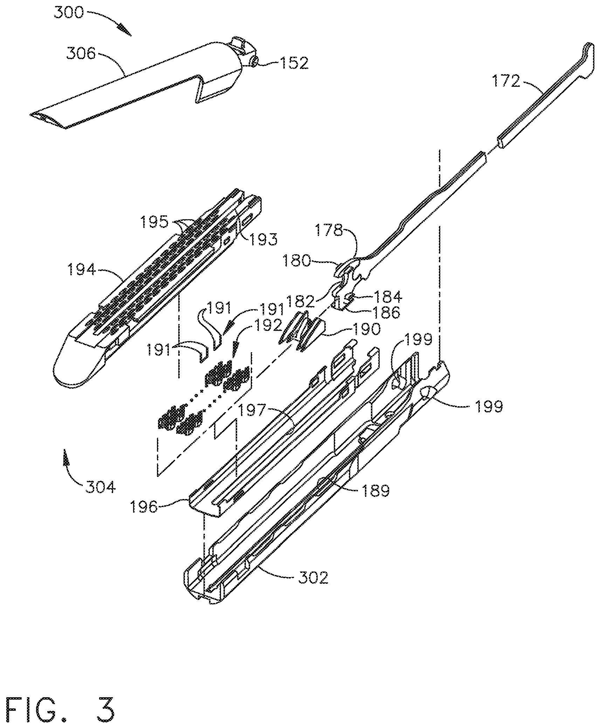

FIG. 3 is an exploded view of one aspect of an end effector 300 of the surgical instrument 10 of FIG. 1 in accordance with one or more aspects of the present disclosure. The end effector 300 may include the anvil 306 and the surgical staple cartridge 304. In this nonlimiting example, the anvil 306 is coupled to an elongated channel 302. For example, apertures 199 can be defined in the elongated channel 302 which can receive pins 152 extending from the anvil 306 and allow the anvil 306 to pivot from an open position to a closed position relative to the elongated channel 302 and surgical staple cartridge 304. A firing bar 172 is configured to longitudinally translate into the end effector 300. The firing bar 172 may be constructed from one solid section, or in various examples, may include a laminate material comprising, for example, a stack of steel plates. The firing bar 172 comprises an E-beam 178 and a cutting edge 182 at a distal end thereof. In various aspects, the E-beam may be referred to as an I-beam. A distally projecting end of the firing bar 172 can be attached to the E-beam 178 element in any suitable manner and can, among other things, assist in spacing the anvil 306 from a surgical staple cartridge 304 positioned in the elongated channel 302 when the anvil 306 is in a closed position. The E-beam 178 also can include a sharpened cutting edge 182 that can be used to sever tissue as the E-beam 178 is advanced distally by the firing bar 172. In operation, the E-beam 178 also can actuate, or fire, the surgical staple cartridge 304. The surgical staple cartridge 304 can include a molded cartridge body 194 that holds a plurality of staples 191 resting upon staple drivers 192 within respective upwardly open staple cavities 195. A wedge sled 190 is driven distally by the E-beam 178, sliding upon a cartridge tray 196 that holds together the various components of the surgical staple cartridge 304. The wedge sled 190 upwardly cams the staple drivers 192 to force out the staples 191 into deforming contact with the anvil 306 while the cutting edge 182 of the E-beam 178 severs clamped tissue.

The E-beam 178 can include upper pins 180 that engage the anvil 306 during firing. The E-beam 178 can further include middle pins 184 and a bottom foot 186 that can engage various portions of the cartridge body 194, cartridge tray 196, and elongated channel 302. When a surgical staple cartridge 304 is positioned within the elongated channel 302, a slot 193 defined in the cartridge body 194 can be aligned with a longitudinal slot 197 defined in the cartridge tray 196 and a slot 189 defined in the elongated channel 302. In use, the E-beam 178 can slide through the aligned longitudinal slots 193, 197, and 189 wherein, as indicated in FIG. 3, the bottom foot 186 of the E-beam 178 can engage a groove running along the bottom surface of elongated channel 302 along the length of slot 189, the middle pins 184 can engage the top surfaces of cartridge tray 196 along the length of longitudinal slot 197, and the upper pins 180 can engage the anvil 306. In such circumstances, the E-beam 178 can space, or limit the relative movement between, the anvil 306 and the surgical staple cartridge 304 as the firing bar 172 is moved distally to fire the staples from the surgical staple cartridge 304 and/or incise the tissue captured between the anvil 306 and the surgical staple cartridge 304. Thereafter, the firing bar 172 and the E-beam 178 can be retracted proximally allowing the anvil 306 to be opened to release the two stapled and severed tissue portions.

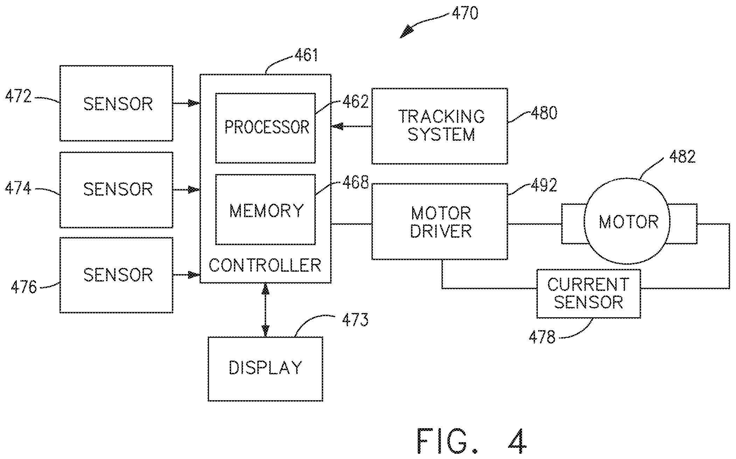

FIG. 4 illustrates a logic diagram of a control system 470 of a surgical instrument or tool in accordance with one or more aspects of the present disclosure. The system 470 comprises a control circuit. The control circuit includes a microcontroller 461 comprising a processor 462 and a memory 468. One or more of sensors 472, 474, 476 , for example, provide real-time feedback to the processor 462. A motor 482, driven by a motor driver 492, operably couples a longitudinally movable displacement member to drive the I-beam knife element. A tracking system 480 is configured to determine the position of the longitudinally movable displacement member. The position information is provided to the processor 462, which can be programmed or configured to determine the position of the longitudinally movable drive member as well as the position of a firing member, firing bar, and I-beam knife element. Additional motors may be provided at the tool driver interface to control I-beam firing, closure tube travel, shaft rotation, and articulation. A display 473 displays a variety of operating conditions of the instruments and may include touch screen functionality for data input. Information displayed on the display 473 may be overlaid with images acquired via endoscopic imaging modules.

In one aspect, the microcontroller 461 may be any single-core or multicore processor such as those known under the trade name ARM Cortex by Texas Instruments. In one aspect, the main microcontroller 461 may be an LM4F230H5QR ARM Cortex-M4F Processor Core, available from Texas Instruments, for example, comprising an on-chip memory of 256 KB single-cycle flash memory, or other non-volatile memory, up to 40 MHz, a prefetch buffer to improve performance above 40 MHz, a 32 KB single-cycle SRAM, and internal ROM loaded with StellarisWare® software, a 2 KB EEPROM, one or more PWM modules, one or more QEI analogs, and/or one or more 12-bit ADCs with 12 analog input channels, details of which are available for the product datasheet.

In one aspect, the microcontroller 461 may comprise a safety controller comprising two controller-based families such as TMS570 and RM4x, known under the trade name Hercules ARM Cortex R4, also by Texas Instruments. The safety controller may be configured specifically for IEC 61508 and ISO 26262 safety critical applications, among others, to provide advanced integrated safety features while delivering scalable performance, connectivity, and memory options.

The microcontroller 461 may be programmed to perform various functions such as precise control over the speed and position of the knife and articulation systems. In one aspect, the microcontroller 461 includes a processor 462 and a memory 468. The electric motor 482 may be a brushed direct current (DC) motor with a gearbox and mechanical links to an articulation or knife system. In one aspect, a motor driver 492 may be an A3941 available from Allegro Microsystems, Inc. Other motor drivers may be readily substituted for use in the tracking system 480 comprising an absolute positioning system. A detailed description of an absolute positioning system is described in U.S. Patent Application Publication No. 2017/0296213, titled SYSTEMS AND METHODS FOR CONTROLLING A SURGICAL STAPLING AND CUTTING INSTRUMENT, which published on October 19, 2017.

The microcontroller 461 may be programmed to provide precise control over the speed and position of displacement members and articulation systems. The microcontroller 461 may be configured to compute a response in the software of the microcontroller 461. The computed response is compared to a measured response of the actual system to obtain an "observed" response, which is used for actual feedback decisions. The observed response is a favorable, tuned value that balances the smooth, continuous nature of the simulated response with the measured response, which can detect outside influences on the system.

In one aspect, the motor 482 may be controlled by the motor driver 492 and can be employed by the firing system of the surgical instrument or tool. In various forms, the motor 482 may be a brushed DC driving motor having a maximum rotational speed of approximately 25,000 RPM. In other arrangements, the motor 482 may include a brushless motor, a cordless motor, a synchronous motor, a stepper motor, or any other suitable electric motor. The motor driver 492 may comprise an H-bridge driver comprising field-effect transistors (FETs), for example. The motor 482 can be powered by a power assembly releasably mounted to the handle assembly or tool housing for supplying control power to the surgical instrument or tool. The power assembly may comprise a battery which may include a number of battery cells connected in series that can be used as the power source to power the surgical instrument or tool. In certain circumstances, the battery cells of the power assembly may be replaceable and/or rechargeable. In at least one example, the battery cells can be lithium-ion batteries which can be couplable to and separable from the power assembly.

The motor driver 492 may be an A3941 available from Allegro Microsystems, Inc. The A3941 492 is a full-bridge controller for use with external N-channel power metal-oxide semiconductor field-effect transistors (MOSFETs) specifically designed for inductive loads, such as brush DC motors. The driver 492 comprises a unique charge pump regulator that provides full (>10 V) gate drive for battery voltages down to 7 V and allows the A3941 to operate with a reduced gate drive, down to 5.5 V. A bootstrap capacitor may be employed to provide the above battery supply voltage required for N-channel MOSFETs. An internal charge pump for the high-side drive allows DC (100% duty cycle) operation. The full bridge can be driven in fast or slow decay modes using diode or synchronous rectification. In the slow decay mode, current recirculation can be through the high-side or the lowside FETs. The power FETs are protected from shoot-through by resistor-adjustable dead time. Integrated diagnostics provide indications of undervoltage, overtemperature, and power bridge faults and can be configured to protect the power MOSFETs under most short circuit conditions. Other motor drivers may be readily substituted for use in the tracking system 480 comprising an absolute positioning system.

The tracking system 480 comprises a controlled motor drive circuit arrangement comprising a position sensor 472 according to one aspect of this disclosure. The position sensor 472 for an absolute positioning system provides a unique position signal corresponding to the location of a displacement member. In one aspect, the displacement member represents a longitudinally movable drive member comprising a rack of drive teeth for meshing engagement with a corresponding drive gear of a gear reducer assembly. In other aspects, the displacement member represents the firing member, which could be adapted and configured to include a rack of drive teeth. In yet another aspect, the displacement member represents a firing bar or the I-beam, each of which can be adapted and configured to include a rack of drive teeth. Accordingly, as used herein, the term displacement member is used generically to refer to any movable member of the surgical instrument or tool such as the drive member, the firing member, the firing bar, the I-beam, or any element that can be displaced. In one aspect, the longitudinally movable drive member is coupled to the firing member, the firing bar, and the I-beam. Accordingly, the absolute positioning system can, in effect, track the linear displacement of the I-beam by tracking the linear displacement of the longitudinally movable drive member. In various other aspects, the displacement member may be coupled to any position sensor 472 suitable for measuring linear displacement. Thus, the longitudinally movable drive member, the firing member, the firing bar, or the I-beam, or combinations thereof, may be coupled to any suitable linear displacement sensor. Linear displacement sensors may include contact or noncontact displacement sensors. Linear displacement sensors may comprise linear variable differential transformers (LVDT), differential variable reluctance transducers (DVRT), a slide potentiometer, a magnetic sensing system comprising a movable magnet and a series of linearly arranged Hall effect sensors, a magnetic sensing system comprising a fixed magnet and a series of movable, linearly arranged Hall effect sensors, an optical sensing system comprising a movable light source and a series of linearly arranged photo diodes or photo detectors, an optical sensing system comprising a fixed light source and a series of movable linearly, arranged photo diodes or photo detectors, or any combination thereof.

The electric motor 482 can include a rotatable shaft that operably interfaces with a gear assembly that is mounted in meshing engagement with a set, or rack, of drive teeth on the displacement member. A sensor element may be operably coupled to a gear assembly such that a single revolution of the position sensor 472 element corresponds to some linear longitudinal translation of the displacement member. An arrangement of gearing and sensors can be connected to the linear actuator, via a rack and pinion arrangement, or a rotary actuator, via a spur gear or other connection. A power source supplies power to the absolute positioning system and an output indicator may display the output of the absolute positioning system. The displacement member represents the longitudinally movable drive member comprising a rack of drive teeth formed thereon for meshing engagement with a corresponding drive gear of the gear reducer assembly. The displacement member represents the longitudinally movable firing member, firing bar, I-beam, or combinations thereof.

A single revolution of the sensor element associated with the position sensor 472 is equivalent to a longitudinal linear displacement d1 of the of the displacement member, where d1 is the longitudinal linear distance that the displacement member moves from point "a" to point "b" after a single revolution of the sensor element coupled to the displacement member. The sensor arrangement may be connected via a gear reduction that results in the position sensor 472 completing one or more revolutions for the full stroke of the displacement member. The position sensor 472 may complete multiple revolutions for the full stroke of the displacement member.

A series of switches, where n is an integer greater than one, may be employed alone or in combination with a gear reduction to provide a unique position signal for more than one revolution of the position sensor 472. The state of the switches are fed back to the microcontroller 461 that applies logic to determine a unique position signal corresponding to the longitudinal linear displacement d1 + d2 + ... dn of the displacement member. The output of the position sensor 472 is provided to the microcontroller 461. The position sensor 472 of the sensor arrangement may comprise a magnetic sensor, an analog rotary sensor like a potentiometer, or an array of analog Hall-effect elements, which output a unique combination of position signals or values.

The position sensor 472 may comprise any number of magnetic sensing elements, such as, for example, magnetic sensors classified according to whether they measure the total magnetic field or the vector components of the magnetic field. The techniques used to produce both types of magnetic sensors encompass many aspects of physics and electronics. The technologies used for magnetic field sensing include search coil, fluxgate, optically pumped, nuclear precession, SQUID, Hall-effect, anisotropic magnetoresistance, giant magnetoresistance, magnetic tunnel junctions, giant magnetoimpedance, magnetostrictive/piezoelectric composites, magnetodiode, magnetotransistor, fiber-optic, magneto-optic, and microelectromechanical systems-based magnetic sensors, among others.

In one aspect, the position sensor 472 for the tracking system 480 comprising an absolute positioning system comprises a magnetic rotary absolute positioning system. The position sensor 472 may be implemented as an AS5055EQFT single-chip magnetic rotary position sensor available from Austria Microsystems, AG. The position sensor 472 is interfaced with the microcontroller 461 to provide an absolute positioning system. The position sensor 472 is a low-voltage and low-power component and includes four Hall-effect elements in an area of the position sensor 472 that is located above a magnet. A high-resolution ADC and a smart power management controller are also provided on the chip. A coordinate rotation digital computer (CORDIC) processor, also known as the digit-by-digit method and Volder's algorithm, is provided to implement a simple and efficient algorithm to calculate hyperbolic and trigonometric functions that require only addition, subtraction, bitshift, and table lookup operations. The angle position, alarm bits, and magnetic field information are transmitted over a standard serial communication interface, such as a serial peripheral interface (SPI) interface, to the microcontroller 461. The position sensor 472 provides 12 or 14 bits of resolution. The position sensor 472 may be an AS5055 chip provided in a small QFN 16-pin 4x4x0.85mm package.

The tracking system 480 comprising an absolute positioning system may comprise and/or be programmed to implement a feedback controller, such as a PID, state feedback, and adaptive controller. A power source converts the signal from the feedback controller into a physical input to the system: in this case the voltage. Other examples include a PWM of the voltage, current, and force. Other sensor(s) may be provided to measure physical parameters of the physical system in addition to the position measured by the position sensor 472. In some aspects, the other sensor(s) can include sensor arrangements such as those described in U.S. Patent No. 9,345,481, titled STAPLE CARTRIDGE TISSUE THICKNESS SENSOR SYSTEM, which issued on May 24, 2016; U.S. Patent Application Publication No. 2014/0263552, titled STAPLE CARTRIDGE TISSUE THICKNESS SENSOR SYSTEM, which published on September 18, 2014; and U.S. Patent Application Serial No. 15/628,175, titled TECHNIQUES FOR ADAPTIVE CONTROL OF MOTOR VELOCITY OF A SURGICAL STAPLING AND CUTTING INSTRUMENT, filed June 20, 2017. In a digital signal processing system, an absolute positioning system is coupled to a digital data acquisition system where the output of the absolute positioning system will have a finite resolution and sampling frequency. The absolute positioning system may comprise a compare-and-combine circuit to combine a computed response with a measured response using algorithms, such as a weighted average and a theoretical control loop, that drive the computed response towards the measured response. The computed response of the physical system takes into account properties like mass, inertial, viscous friction, inductance resistance, etc., to predict what the states and outputs of the physical system will be by knowing the input.

The absolute positioning system provides an absolute position of the displacement member upon power-up of the instrument, without retracting or advancing the displacement member to a reset (zero or home) position as may be required with conventional rotary encoders that merely count the number of steps forwards or backwards that the motor 482 has taken to infer the position of a device actuator, drive bar, knife, or the like.

A sensor 474, such as, for example, a strain gauge or a micro-strain gauge, is configured to measure one or more parameters of the end effector, such as, for example, the amplitude of the strain exerted on the anvil during a clamping operation, which can be indicative of the closure forces applied to the anvil. The measured strain is converted to a digital signal and provided to the processor 462. Alternatively, or in addition to the sensor 474, a sensor 476, such as, for example, a load sensor, can measure the closure force applied by the closure drive system to the anvil. The sensor 476, such as, for example, a load sensor, can measure the firing force applied to an I-beam in a firing stroke of the surgical instrument or tool. The I-beam is configured to engage a wedge sled, which is configured to upwardly cam staple drivers to force out staples into deforming contact with an anvil. The I-beam also includes a sharpened cutting edge that can be used to sever tissue as the I-beam is advanced distally by the firing bar. Alternatively, a current sensor 478 can be employed to measure the current drawn by the motor 482. The force required to advance the firing member can correspond to the current drawn by the motor 482, for example. The measured force is converted to a digital signal and provided to the processor 462.

In one form, the strain gauge sensor 474 can be used to measure the force applied to the tissue by the end effector. A strain gauge can be coupled to the end effector to measure the force on the tissue being treated by the end effector. A system for measuring forces applied to the tissue grasped by the end effector comprises a strain gauge sensor 474, such as, for example, a micro-strain gauge, that is configured to measure one or more parameters of the end effector, for example. In one aspect, the strain gauge sensor 474 can measure the amplitude or magnitude of the strain exerted on a jaw member of an end effector during a clamping operation, which can be indicative of the tissue compression. The measured strain is converted to a digital signal and provided to a processor 462 of the microcontroller 461. A load sensor 476 can measure the force used to operate the knife element, for example, to cut the tissue captured between the anvil and the staple cartridge. A magnetic field sensor can be employed to measure the thickness of the captured tissue. The measurement of the magnetic field sensor also may be converted to a digital signal and provided to the processor 462.

The measurements of the tissue compression, the tissue thickness, and/or the force required to close the end effector on the tissue, as respectively measured by the sensors 474, 476, can be used by the microcontroller 461 to characterize the selected position of the firing member and/or the corresponding value of the speed of the firing member. In one instance, a memory 468 may store a technique, an equation, and/or a lookup table which can be employed by the microcontroller 461 in the assessment.

The control system 470 of the surgical instrument or tool also may comprise wired or wireless communication circuits to communicate with a modular communication hub, for example, as discussed in U.S. Patent Application Serial No. 15/940,632, entitled DATA STRIPPING METHOD TO INTERROGATE PATIENT RECORDS AND CREATE ANONYMIZED RECORD; Attorney Docket No. END8500USNP/170767.

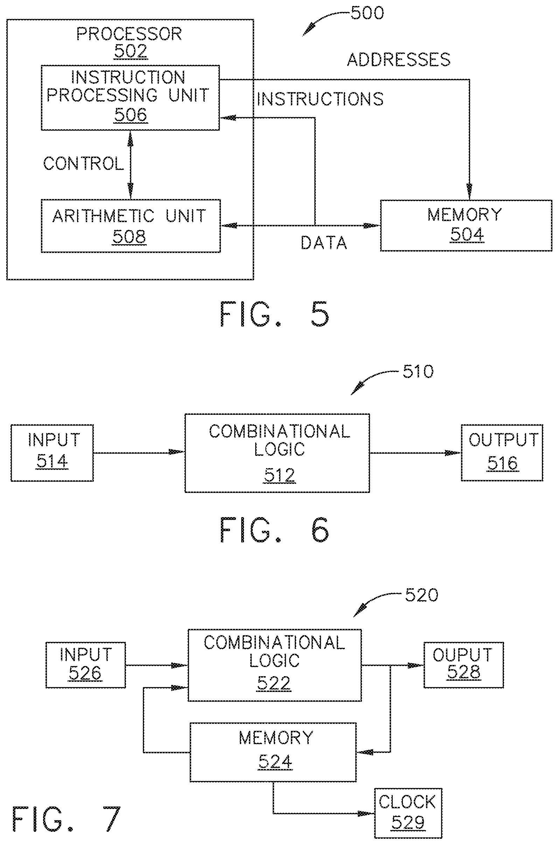

FIG. 5 illustrates a control circuit 500 configured to control aspects of the surgical instrument or tool according to one aspect of this disclosure. The control circuit 500 can be configured to implement various processes described herein. The control circuit 500 may comprise a microcontroller comprising one or more processors 502 (e.g., microprocessor, microcontroller) coupled to at least one memory circuit 504. The memory circuit 504 stores machine-executable instructions that, when executed by the processor 502, cause the processor 502 to execute machine instructions to implement various processes described herein. The processor 502 may be any one of a number of single-core or multicore processors known in the art. The memory circuit 504 may comprise volatile and non-volatile storage media. The processor 502 may include an instruction processing unit 506 and an arithmetic unit 508. The instruction processing unit may be configured to receive instructions from the memory circuit 504 of this disclosure.

FIG. 6 illustrates a combinational logic circuit 510 configured to control aspects of the surgical instrument or tool according to one aspect of this disclosure. The combinational logic circuit 510 can be configured to implement various processes described herein. The combinational logic circuit 510 may comprise a finite state machine comprising a combinational logic 512 configured to receive data associated with the surgical instrument or tool at an input 514, process the data by the combinational logic 512, and provide an output 516.

FIG. 7 illustrates a sequential logic circuit 520 configured to control aspects of the surgical instrument or tool according to one aspect of this disclosure. The sequential logic circuit 520 or the combinational logic 522 can be configured to implement various processes described herein. The sequential logic circuit 520 may comprise a finite state machine. The sequential logic circuit 520 may comprise a combinational logic 522, at least one memory circuit 524, and a clock 529, for example. The at least one memory circuit 524 can store a current state of the finite state machine. In certain instances, the sequential logic circuit 520 may be synchronous or asynchronous. The combinational logic 522 is configured to receive data associated with the surgical instrument or tool from an input 526, process the data by the combinational logic 522, and provide an output 528. In other aspects, the circuit may comprise a combination of a processor (e.g., processor 502, FIG. 5) and a finite state machine to implement various processes herein. In other aspects, the finite state machine may comprise a combination of a combinational logic circuit (e.g., combinational logic circuit 510, FIG. 6) and the sequential logic circuit 520.

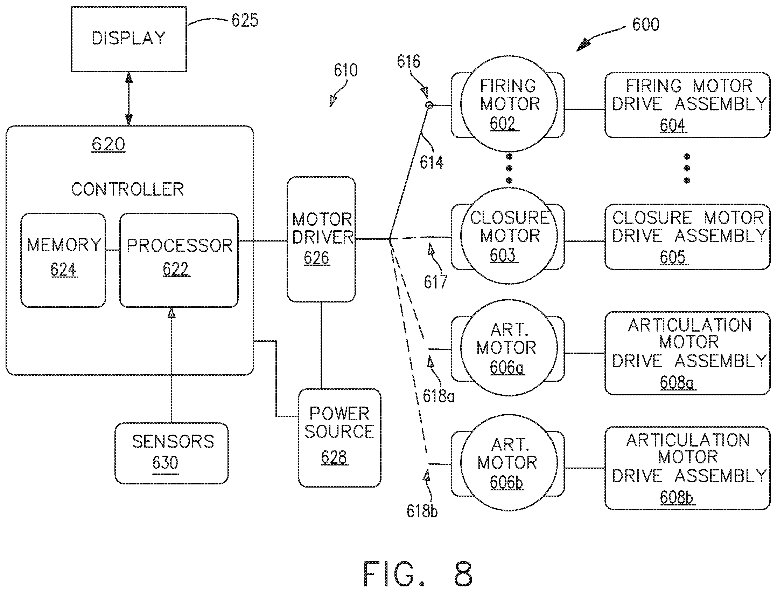

FIG. 8 illustrates a surgical instrument or tool comprising a plurality of motors which can be activated to perform various functions. In certain instances, a first motor can be activated to perform a first function, a second motor can be activated to perform a second function, a third motor can be activated to perform a third function, a fourth motor can be activated to perform a fourth function, and so on. In certain instances, the plurality of motors of robotic surgical instrument 600 can be individually activated to cause firing, closure, and/or articulation motions in the end effector. The firing, closure, and/or articulation motions can be transmitted to the end effector through a shaft assembly, for example.

In certain instances, the surgical instrument system or tool may include a firing motor 602. The firing motor 602 may be operably coupled to a firing motor drive assembly 604 which can be configured to transmit firing motions, generated by the motor 602 to the end effector, in particular to displace the I-beam element. In certain instances, the firing motions generated by the motor 602 may cause the staples to be deployed from the staple cartridge into tissue captured by the end effector and/or the cutting edge of the I-beam element to be advanced to cut the captured tissue, for example. The I-beam element may be retracted by reversing the direction of the motor 602.

In certain instances, the surgical instrument or tool may include a closure motor 603. The closure motor 603 may be operably coupled to a closure motor drive assembly 605 which can be configured to transmit closure motions, generated by the motor 603 to the end effector, in particular to displace a closure tube to close the anvil and compress tissue between the anvil and the staple cartridge. The closure motions may cause the end effector to transition from an open configuration to an approximated configuration to capture tissue, for example. The end effector may be transitioned to an open position by reversing the direction of the motor 603.

In certain instances, the surgical instrument or tool may include one or more articulation motors 606a, 606b, for example. The motors 606a, 606b may be operably coupled to respective articulation motor drive assemblies 608a, 608b, which can be configured to transmit articulation motions generated by the motors 606a, 606b to the end effector. In certain instances, the articulation motions may cause the end effector to articulate relative to the shaft, for example.

As described above, the surgical instrument or tool may include a plurality of motors which may be configured to perform various independent functions. In certain instances, the plurality of motors of the surgical instrument or tool can be individually or separately activated to perform one or more functions while the other motors remain inactive. For example, the articulation motors 606a, 606b can be activated to cause the end effector to be articulated while the firing motor 602 remains inactive. Alternatively, the firing motor 602 can be activated to fire the plurality of staples, and/or to advance the cutting edge, while the articulation motor 606 remains inactive. Furthermore the closure motor 603 may be activated simultaneously with the firing motor 602 to cause the closure tube and the I-beam element to advance distally as described in more detail hereinbelow.

In certain instances, the surgical instrument or tool may include a common control module 610 which can be employed with a plurality of motors of the surgical instrument or tool. In certain instances, the common control module 610 may accommodate one of the plurality of motors at a time. For example, the common control module 610 can be couplable to and separable from the plurality of motors of the robotic surgical instrument individually. In certain instances, a plurality of the motors of the surgical instrument or tool may share one or more common control modules such as the common control module 610. In certain instances, a plurality of motors of the surgical instrument or tool can be individually and selectively engaged with the common control module 610. In certain instances, the common control module 610 can be selectively switched from interfacing with one of a plurality of motors of the surgical instrument or tool to interfacing with another one of the plurality of motors of the surgical instrument or tool.

In at least one example, the common control module 610 can be selectively switched between operable engagement with the articulation motors 606a, 606b and operable engagement with either the firing motor 602 or the closure motor 603. In at least one example, as illustrated in FIG. 8, a switch 614 can be moved or transitioned between a plurality of positions and/or states. In a first position 616, the switch 614 may electrically couple the common control module 610 to the firing motor 602; in a second position 617, the switch 614 may electrically couple the common control module 610 to the closure motor 603; in a third position 618a, the switch 614 may electrically couple the common control module 610 to the first articulation motor 606a; and in a fourth position 618b, the switch 614 may electrically couple the common control module 610 to the second articulation motor 606b, for example. In certain instances, separate common control modules 610 can be electrically coupled to the firing motor 602, the closure motor 603, and the articulations motor 606a, 606b at the same time. In certain instances, the switch 614 may be a mechanical switch, an electromechanical switch, a solid-state switch, or any suitable switching mechanism.

Each of the motors 602, 603, 606a, 606b may comprise a torque sensor to measure the output torque on the shaft of the motor. The force on an end effector may be sensed in any conventional manner, such as by force sensors on the outer sides of the jaws or by a torque sensor for the motor actuating the jaws.

In various instances, as illustrated in FIG. 8, the common control module 610 may comprise a motor driver 626 which may comprise one or more H-Bridge FETs. The motor driver 626 may modulate the power transmitted from a power source 628 to a motor coupled to the common control module 610 based on input from a microcontroller 620 (the "controller"), for example. In certain instances, the microcontroller 620 can be employed to determine the current drawn by the motor, for example, while the motor is coupled to the common control module 610, as described above.

In certain instances, the microcontroller 620 may include a microprocessor 622 (the "processor") and one or more non-transitory computer-readable mediums or memory units 624 (the "memory"). In certain instances, the memory 624 may store various program instructions, which when executed may cause the processor 622 to perform a plurality of functions and/or calculations described herein. In certain instances, one or more of the memory units 624 may be coupled to the processor 622, for example.

In certain instances, the power source 628 can be employed to supply power to the microcontroller 620, for example. In certain instances, the power source 628 may comprise a battery (or "battery pack" or "power pack"), such as a lithium-ion battery, for example. In certain instances, the battery pack may be configured to be releasably mounted to a handle for supplying power to the surgical instrument 600. A number of battery cells connected in series may be used as the power source 628. In certain instances, the power source 628 may be replaceable and/or rechargeable, for example.

In various instances, the processor 622 may control the motor driver 626 to control the position, direction of rotation, and/or velocity of a motor that is coupled to the common control module 610. In certain instances, the processor 622 can signal the motor driver 626 to stop and/or disable a motor that is coupled to the common control module 610. It should be understood that the term "processor" as used herein includes any suitable microprocessor, microcontroller, or other basic computing device that incorporates the functions of a computer's central processing unit (CPU) on an integrated circuit or, at most, a few integrated circuits. The processor is a multipurpose, programmable device that accepts digital data as input, processes it according to instructions stored in its memory, and provides results as output. It is an example of sequential digital logic, as it has internal memory. Processors operate on numbers and symbols represented in the binary numeral system.

In one instance, the processor 622 may be any single-core or multicore processor such as those known under the trade name ARM Cortex by Texas Instruments. In certain instances, the microcontroller 620 may be an LM 4F230H5QR, available from Texas Instruments, for example. In at least one example, the Texas Instruments LM4F230H5QR is an ARM Cortex-M4F Processor Core comprising an on-chip memory of 256 KB single-cycle flash memory, or other non-volatile memory, up to 40 MHz, a prefetch buffer to improve performance above 40 MHz, a 32 KB single-cycle SRAM, an internal ROM loaded with StellarisWare® software, a 2 KB EEPROM, one or more PWM modules, one or more QEI analogs, one or more 12-bit ADCs with 12 analog input channels, among other features that are readily available for the product datasheet. Other microcontrollers may be readily substituted for use with the module 4410. Accordingly, the present disclosure should not be limited in this context.

In certain instances, the memory 624 may include program instructions for controlling each of the motors of the surgical instrument 600 that are couplable to the common control module 610. For example, the memory 624 may include program instructions for controlling the firing motor 602, the closure motor 603, and the articulation motors 606a, 606b. Such program instructions may cause the processor 622 to control the firing, closure, and articulation functions in accordance with inputs from algorithms or control programs of the surgical instrument or tool.

In certain instances, one or more mechanisms and/or sensors such as, for example, sensors 630 can be employed to alert the processor 622 to the program instructions that should be used in a particular setting. For example, the sensors 630 may alert the processor 622 to use the program instructions associated with firing, closing, and articulating the end effector. In certain instances, the sensors 630 may comprise position sensors which can be employed to sense the position of the switch 614, for example. Accordingly, the processor 622 may use the program instructions associated with firing the I-beam of the end effector upon detecting, through the sensors 630 for example, that the switch 614 is in the first position 616; the processor 622 may use the program instructions associated with closing the anvil upon detecting, through the sensors 630 for example, that the switch 614 is in the second position 617; and the processor 622 may use the program instructions associated with articulating the end effector upon detecting, through the sensors 630 for example, that the switch 614 is in the third or fourth position 618a, 618b.

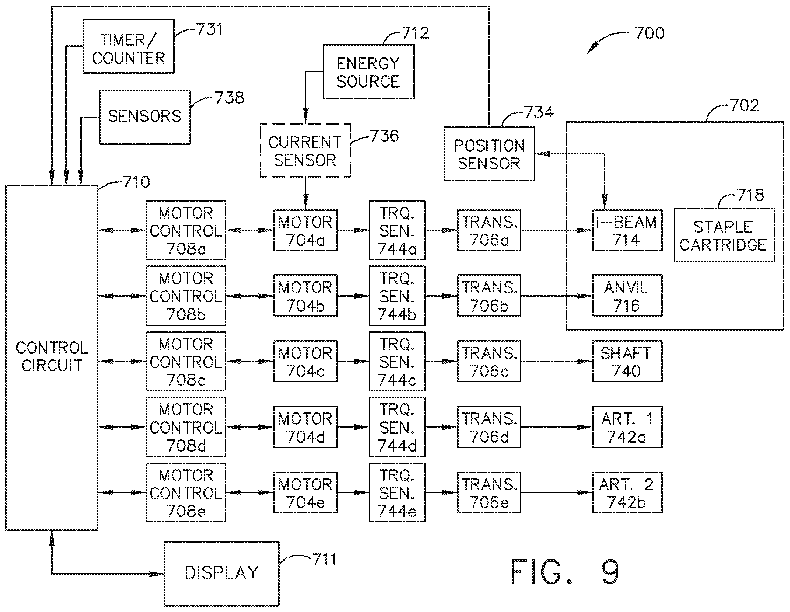

FIG. 9 is a schematic diagram of a robotic surgical instrument 700 configured to operate a surgical tool described herein according to one aspect of this disclosure. The robotic surgical instrument 700 may be programmed or configured to control distal/proximal translation of a displacement member, distal/proximal displacement of a closure tube, shaft rotation, and articulation, either with single or multiple articulation drive links. In one aspect, the surgical instrument 700 may be programmed or configured to individually control a firing member, a closure member, a shaft member, and/or one or more articulation members. The surgical instrument 700 comprises a control circuit 710 configured to control motor-driven firing members, closure members, shaft members, and/or one or more articulation members.

In one aspect, the robotic surgical instrument 700 comprises a control circuit 710 configured to control an anvil 716 and an I-beam 714 (including a sharp cutting edge) portion of an end effector 702, a removable staple cartridge 718, a shaft 740, and one or more articulation members 742a, 742b via a plurality of motors 704a-704e. A position sensor 734 may be configured to provide position feedback of the I-beam 714 to the control circuit 710. Other sensors 738 may be configured to provide feedback to the control circuit 710. A timer/counter 731 provides timing and counting information to the control circuit 710. An energy source 712 may be provided to operate the motors 704a-704e, and a current sensor 736 provides motor current feedback to the control circuit 710. The motors 704a-704e can be operated individually by the control circuit 710 in an 718 open-loop or closed-loop feedback control.

In one aspect, the control circuit 710 may comprise one or more microcontrollers, microprocessors, or other suitable processors for executing instructions that cause the processor or processors to perform one or more tasks. In one aspect, a timer/counter 731 provides an output signal, such as the elapsed time or a digital count, to the control circuit 710 to correlate the position of the I-beam 714 as determined by the position sensor 734 with the output of the timer/counter 731 such that the control circuit 710 can determine the position of the I-beam 714 at a specific time (t) relative to a starting position or the time (t) when the I-beam 714 is at a specific position relative to a starting position. The timer/counter 731 may be configured to measure elapsed time, count external events, or time external events.

In one aspect, the control circuit 710 may be programmed to control functions of the end effector 702 based on one or more tissue conditions. The control circuit 710 may be programmed to sense tissue conditions, such as thickness, either directly or indirectly, as described herein. The control circuit 710 may be programmed to select a firing control program or closure control program based on tissue conditions. A firing control program may describe the distal motion of the displacement member. Different firing control programs may be selected to better treat different tissue conditions. For example, when thicker tissue is present, the control circuit 710 may be programmed to translate the displacement member at a lower velocity and/or with lower power. When thinner tissue is present, the control circuit 710 may be programmed to translate the displacement member at a higher velocity and/or with higher power. A closure control program may control the closure force applied to the tissue by the anvil 716. Other control programs control the rotation of the shaft 740 and the articulation members 742a, 742b.

In one aspect, the control circuit 710 may generate motor set point signals. The motor set point signals may be provided to various motor controllers 708a-708e. The motor controllers 708a-708e may comprise one or more circuits configured to provide motor drive signals to the motors 704a-704e to drive the motors 704a-704e as described herein. In some examples, the motors 704a-704e may be brushed DC electric motors. For example, the velocity of the motors 704a-704e may be proportional to the respective motor drive signals. In some examples, the motors 704a-704e may be brushless DC electric motors, and the respective motor drive signals may comprise a PWM signal provided to one or more stator windings of the motors 704a-704e. Also, in some examples, the motor controllers 708a-708e may be omitted and the control circuit 710 may generate the motor drive signals directly.

In one aspect, the control circuit 710 may initially operate each of the motors 704a-704e in an open-loop configuration for a first open-loop portion of a stroke of the displacement member. Based on the response of the robotic surgical instrument 700 during the open-loop portion of the stroke, the control circuit 710 may select a firing control program in a closed-loop configuration. The response of the instrument may include a translation distance of the displacement member during the open-loop portion, a time elapsed during the open-loop portion, the energy provided to one of the motors 704a-704e during the open-loop portion, a sum of pulse widths of a motor drive signal, etc. After the open-loop portion, the control circuit 710 may implement the selected firing control program for a second portion of the displacement member stroke. For example, during a closed-loop portion of the stroke, the control circuit 710 may modulate one of the motors 704a-704e based on translation data describing a position of the displacement member in a closed-loop manner to translate the displacement member at a constant velocity.

In one aspect, the motors 704a-704e may receive power from an energy source 712. The energy source 712 may be a DC power supply driven by a main alternating current power source, a battery, a super capacitor, or any other suitable energy source. The motors 704a-704e may be mechanically coupled to individual movable mechanical elements such as the I-beam 714, anvil 716, shaft 740, articulation 742a, and articulation 742b via respective transmissions 706a-706e. The transmissions 706a-706e may include one or more gears or other linkage components to couple the motors 704a-704e to movable mechanical elements. A position sensor 734 may sense a position of the I-beam 714. The position sensor 734 may be or include any type of sensor that is capable of generating position data that indicate a position of the I-beam 714. In some examples, the position sensor 734 may include an encoder configured to provide a series of pulses to the control circuit 710 as the I-beam 714 translates distally and proximally. The control circuit 710 may track the pulses to determine the position of the I-beam 714. Other suitable position sensors may be used, including, for example, a proximity sensor. Other types of position sensors may provide other signals indicating motion of the I-beam 714. Also, in some examples, the position sensor 734 may be omitted. Where any of the motors 704a-704e is a stepper motor, the control circuit 710 may track the position of the I-beam 714 by aggregating the number and direction of steps that the motor 704 has been instructed to execute. The position sensor 734 may be located in the end effector 702 or at any other portion of the instrument. The outputs of each of the motors 704a-704e include a torque sensor 744a-744e to sense force and have an encoder to sense rotation of the drive shaft.

In one aspect, the control circuit 710 is configured to drive a firing member such as the I-beam 714 portion of the end effector 702. The control circuit 710 provides a motor set point to a motor control 708a, which provides a drive signal to the motor 704a. The output shaft of the motor 704a is coupled to a torque sensor 744a. The torque sensor 744a is coupled to a transmission 706a which is coupled to the I-beam 714. The transmission 706a comprises movable mechanical elements such as rotating elements and a firing member to control the movement of the I-beam 714 distally and proximally along a longitudinal axis of the end effector 702. In one aspect, the motor 704a may be coupled to the knife gear assembly, which includes a knife gear reduction set that includes a first knife drive gear and a second knife drive gear. A torque sensor 744a provides a firing force feedback signal to the control circuit 710. The firing force signal represents the force required to fire or displace the I-beam 714. A position sensor 734 may be configured to provide the position of the I-beam 714 along the firing stroke or the position of the firing member as a feedback signal to the control circuit 710. The end effector 702 may include additional sensors 738 configured to provide feedback signals to the control circuit 710. When ready to use, the control circuit 710 may provide a firing signal to the motor control 708a. In response to the firing signal, the motor 704a may drive the firing member distally along the longitudinal axis of the end effector 702 from a proximal stroke start position to a stroke end position distal to the stroke start position. As the firing member translates distally, an I-beam 714, with a cutting element positioned at a distal end, advances distally to cut tissue located between the staple cartridge 718 and the anvil 716.

In one aspect, the control circuit 710 is configured to drive a closure member such as the anvil 716 portion of the end effector 702. The control circuit 710 provides a motor set point to a motor control 708b, which provides a drive signal to the motor 704b. The output shaft of the motor 704b is coupled to a torque sensor 744b. The torque sensor 744b is coupled to a transmission 706b which is coupled to the anvil 716. The transmission 706b comprises movable mechanical elements such as rotating elements and a closure member to control the movement of the anvil 716 from the open and closed positions. In one aspect, the motor 704b is coupled to a closure gear assembly, which includes a closure reduction gear set that is supported in meshing engagement with the closure spur gear. The torque sensor 744b provides a closure force feedback signal to the control circuit 710. The closure force feedback signal represents the closure force applied to the anvil 716. The position sensor 734 may be configured to provide the position of the closure member as a feedback signal to the control circuit 710. Additional sensors 738 in the end effector 702 may provide the closure force feedback signal to the control circuit 710. The pivotable anvil 716 is positioned opposite the staple cartridge 718. When ready to use, the control circuit 710 may provide a closure signal to the motor control 708b. In response to the closure signal, the motor 704b advances a closure member to grasp tissue between the anvil 716 and the staple cartridge 718.

In one aspect, the control circuit 710 is configured to rotate a shaft member such as the shaft 740 to rotate the end effector 702. The control circuit 710 provides a motor set point to a motor control 708c, which provides a drive signal to the motor 704c. The output shaft of the motor 704c is coupled to a torque sensor 744c. The torque sensor 744c is coupled to a transmission 706c which is coupled to the shaft 740. The transmission 706c comprises movable mechanical elements such as rotating elements to control the rotation of the shaft 740 clockwise or counterclockwise up to and over 360°. In one aspect, the motor 704c is coupled to the rotational transmission assembly, which includes a tube gear segment that is formed on (or attached to) the proximal end of the proximal closure tube for operable engagement by a rotational gear assembly that is operably supported on the tool mounting plate. The torque sensor 744c provides a rotation force feedback signal to the control circuit 710. The rotation force feedback signal represents the rotation force applied to the shaft 740. The position sensor 734 may be configured to provide the position of the closure member as a feedback signal to the control circuit 710. Additional sensors 738 such as a shaft encoder may provide the rotational position of the shaft 740 to the control circuit 710.

In one aspect, the control circuit 710 is configured to articulate the end effector 702. The control circuit 710 provides a motor set point to a motor control 708d, which provides a drive signal to the motor 704d. The output shaft of the motor 704d is coupled to a torque sensor 744d. The torque sensor 744d is coupled to a transmission 706d which is coupled to an articulation member 742a. The transmission 706d comprises movable mechanical elements such as articulation elements to control the articulation of the end effector 702 ±65°. In one aspect, the motor 704d is coupled to an articulation nut, which is rotatably journaled on the proximal end portion of the distal spine portion and is rotatably driven thereon by an articulation gear assembly. The torque sensor 744d provides an articulation force feedback signal to the control circuit 710. The articulation force feedback signal represents the articulation force applied to the end effector 702. Sensors 738, such as an articulation encoder, may provide the articulation position of the end effector 702 to the control circuit 710.

In another aspect, the articulation function of the robotic surgical system 700 may comprise two articulation members, or links, 742a, 742b. These articulation members 742a, 742b are driven by separate disks on the robot interface (the rack) which are driven by the two motors 708d, 708e. When the separate firing motor 704a is provided, each of articulation links 742a, 742b can be antagonistically driven with respect to the other link in order to provide a resistive holding motion and a load to the head when it is not moving and to provide an articulation motion as the head is articulated. The articulation members 742a, 742b attach to the head at a fixed radius as the head is rotated. Accordingly, the mechanical advantage of the push-and-pull link changes as the head is rotated. This change in the mechanical advantage may be more pronounced with other articulation link drive systems.

In one aspect, the one or more motors 704a-704e may comprise a brushed DC motor with a gearbox and mechanical links to a firing member, closure member, or articulation member. Another example includes electric motors 704a-704e that operate the movable mechanical elements such as the displacement member, articulation links, closure tube, and shaft. An outside influence is an unmeasured, unpredictable influence of things like tissue, surrounding bodies, and friction on the physical system. Such outside influence can be referred to as drag, which acts in opposition to one of electric motors 704a-704e. The outside influence, such as drag, may cause the operation of the physical system to deviate from a desired operation of the physical system.

In one aspect, the position sensor 734 may be implemented as an absolute positioning system. In one aspect, the position sensor 734 may comprise a magnetic rotary absolute positioning system implemented as an AS5055EQFT single-chip magnetic rotary position sensor available from Austria Microsystems, AG. The position sensor 734 may interface with the control circuit 710 to provide an absolute positioning system. The position may include multiple Hall-effect elements located above a magnet and coupled to a CORDIC processor, also known as the digit-by-digit method and Volder's algorithm, that is provided to implement a simple and efficient algorithm to calculate hyperbolic and trigonometric functions that require only addition, subtraction, bitshift, and table lookup operations.

In one aspect, the control circuit 710 may be in communication with one or more sensors 738. The sensors 738 may be positioned on the end effector 702 and adapted to operate with the robotic surgical instrument 700 to measure the various derived parameters such as the gap distance versus time, tissue compression versus time, and anvil strain versus time. The sensors 738 may comprise a magnetic sensor, a magnetic field sensor, a strain gauge, a load cell, a pressure sensor, a force sensor, a torque sensor, an inductive sensor such as an eddy current sensor, a resistive sensor, a capacitive sensor, an optical sensor, and/or any other suitable sensor for measuring one or more parameters of the end effector 702. The sensors 738 may include one or more sensors. The sensors 738 may be located on the staple cartridge 718 deck to determine tissue location using segmented electrodes. The torque sensors 744a-744e may be configured to sense force such as firing force, closure force, and/or articulation force, among others. Accordingly, the control circuit 710 can sense (1) the closure load experienced by the distal closure tube and its position, (2) the firing member at the rack and its position, (3) what portion of the staple cartridge 718 has tissue on it, and (4) the load and position on both articulation rods.

In one aspect, the one or more sensors 738 may comprise a strain gauge, such as a micro-strain gauge, configured to measure the magnitude of the strain in the anvil 716 during a clamped condition. The strain gauge provides an electrical signal whose amplitude varies with the magnitude of the strain. The sensors 738 may comprise a pressure sensor configured to detect a pressure generated by the presence of compressed tissue between the anvil 716 and the staple cartridge 718. The sensors 738 may be configured to detect impedance of a tissue section located between the anvil 716 and the staple cartridge 718 that is indicative of the thickness and/or fullness of tissue located therebetween.

In one aspect, the sensors 738 may be implemented as one or more limit switches, electromechanical devices, solid-state switches, Hall-effect devices, magneto-resistive (MR) devices, giant magneto-resistive (GMR) devices, magnetometers, among others. In other implementations, the sensors 738 may be implemented as solid-state switches that operate under the influence of light, such as optical sensors, IR sensors, ultraviolet sensors, among others. Still, the switches may be solid-state devices such as transistors (e.g., FET, junction FET, MOSFET, bipolar, and the like). In other implementations, the sensors 738 may include electrical conductorless switches, ultrasonic switches, accelerometers, and inertial sensors, among others.

In one aspect, the sensors 738 may be configured to measure forces exerted on the anvil 716 by the closure drive system. For example, one or more sensors 738 can be at an interaction point between the closure tube and the anvil 716 to detect the closure forces applied by the closure tube to the anvil 716. The forces exerted on the anvil 716 can be representative of the tissue compression experienced by the tissue section captured between the anvil 716 and the staple cartridge 718. The one or more sensors 738 can be positioned at various interaction points along the closure drive system to detect the closure forces applied to the anvil 716 by the closure drive system. The one or more sensors 738 may be sampled in real time during a clamping operation by the processor of the control circuit 710. The control circuit 710 receives real-time sample measurements to provide and analyze time-based information and assess, in real time, closure forces applied to the anvil 716.

In one aspect, a current sensor 736 can be employed to measure the current drawn by each of the motors 704a-704e. The force required to advance any of the movable mechanical elements such as the I-beam 714 corresponds to the current drawn by one of the motors 704a-704e. The force is converted to a digital signal and provided to the control circuit 710. The control circuit 710 can be configured to simulate the response of the actual system of the instrument in the software of the controller. A displacement member can be actuated to move an I-beam 714 in the end effector 702 at or near a target velocity. The robotic surgical instrument 700 can include a feedback controller, which can be one of any feedback controllers, including, but not limited to a PID, a state feedback, a linear-quadratic (LQR), and/or an adaptive controller, for example. The robotic surgical instrument 700 can include a power source to convert the signal from the feedback controller into a physical input such as case voltage, PWM voltage, frequency modulated voltage, current, torque, and/or force, for example. Additional details are disclosed in U.S. Patent Application Serial No. 15/636,829, titled CLOSED LOOP VELOCITY CONTROL TECHNIQUES FOR ROBOTIC SURGICAL INSTRUMENT, filed June 29, 2017.

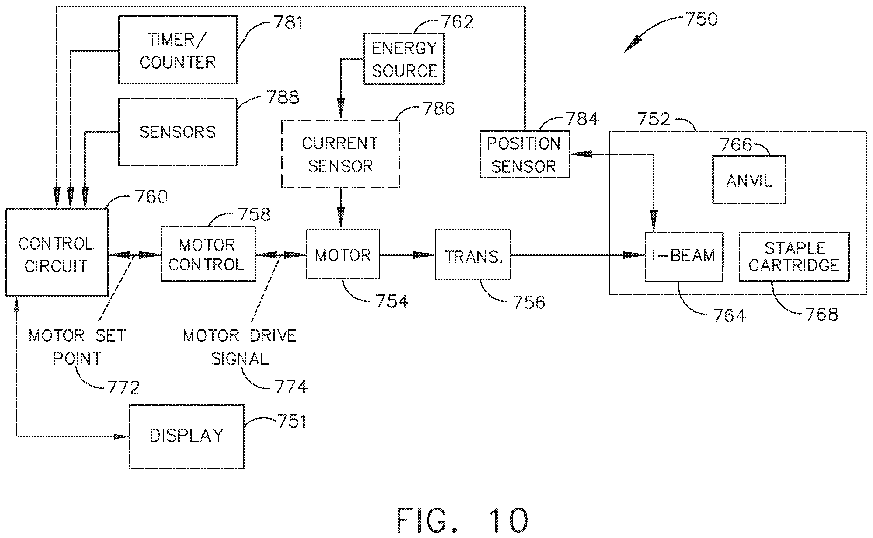

FIG. 10 illustrates a block diagram of a surgical instrument 750 programmed to control the distal translation of a displacement member according to one aspect of this disclosure. In one aspect, the surgical instrument 750 is programmed to control the distal translation of a displacement member such as the I-beam 764. The surgical instrument 750 comprises an end effector 752 that may comprise an anvil 766, an I-beam 764 (including a sharp cutting edge), and a removable staple cartridge 768.

The position, movement, displacement, and/or translation of a linear displacement member, such as the I-beam 764, can be measured by an absolute positioning system, sensor arrangement, and position sensor 784. Because the I-beam 764 is coupled to a longitudinally movable drive member, the position of the I-beam 764 can be determined by measuring the position of the longitudinally movable drive member employing the position sensor 784. Accordingly, in the following description, the position, displacement, and/or translation of the I-beam 764 can be achieved by the position sensor 784 as described herein. A control circuit 760 may be programmed to control the translation of the displacement member, such as the I-beam 764. The control circuit 760, in some examples, may comprise one or more microcontrollers, microprocessors, or other suitable processors for executing instructions that cause the processor or processors to control the displacement member, e.g., the I-beam 764, in the manner described. In one aspect, a timer/counter 781 provides an output signal, such as the elapsed time or a digital count, to the control circuit 760 to correlate the position of the I-beam 764 as determined by the position sensor 784 with the output of the timer/counter 781 such that the control circuit 760 can determine the position of the I-beam 764 at a specific time (t) relative to a starting position. The timer/counter 781 may be configured to measure elapsed time, count external events, or time external events.

The control circuit 760 may generate a motor set point signal 772. The motor set point signal 772 may be provided to a motor controller 758. The motor controller 758 may comprise one or more circuits configured to provide a motor drive signal 774 to the motor 754 to drive the motor 754 as described herein. In some examples, the motor 754 may be a brushed DC electric motor. For example, the velocity of the motor 754 may be proportional to the motor drive signal 774. In some examples, the motor 754 may be a brushless DC electric motor and the motor drive signal 774 may comprise a PWM signal provided to one or more stator windings of the motor 754. Also, in some examples, the motor controller 758 may be omitted, and the control circuit 760 may generate the motor drive signal 774 directly.

The motor 754 may receive power from an energy source 762. The energy source 762 may be or include a battery, a super capacitor, or any other suitable energy source. The motor 754 may be mechanically coupled to the I-beam 764 via a transmission 756. The transmission 756 may include one or more gears or other linkage components to couple the motor 754 to the I-beam 764. A position sensor 784 may sense a position of the I-beam 764. The position sensor 784 may be or include any type of sensor that is capable of generating position data that indicate a position of the I-beam 764. In some examples, the position sensor 784 may include an encoder configured to provide a series of pulses to the control circuit 760 as the I-beam 764 translates distally and proximally. The control circuit 760 may track the pulses to determine the position of the I-beam 764. Other suitable position sensors may be used, including, for example, a proximity sensor. Other types of position sensors may provide other signals indicating motion of the I-beam 764. Also, in some examples, the position sensor 784 may be omitted. Where the motor 754 is a stepper motor, the control circuit 760 may track the position of the I-beam 764 by aggregating the number and direction of steps that the motor 754 has been instructed to execute. The position sensor 784 may be located in the end effector 752 or at any other portion of the instrument.

The control circuit 760 may be in communication with one or more sensors 788. The sensors 788 may be positioned on the end effector 752 and adapted to operate with the surgical instrument 750 to measure the various derived parameters such as gap distance versus time, tissue compression versus time, and anvil strain versus time. The sensors 788 may comprise a magnetic sensor, a magnetic field sensor, a strain gauge, a pressure sensor, a force sensor, an inductive sensor such as an eddy current sensor, a resistive sensor, a capacitive sensor, an optical sensor, and/or any other suitable sensor for measuring one or more parameters of the end effector 752. The sensors 788 may include one or more sensors.

The one or more sensors 788 may comprise a strain gauge, such as a micro-strain gauge, configured to measure the magnitude of the strain in the anvil 766 during a clamped condition. The strain gauge provides an electrical signal whose amplitude varies with the magnitude of the strain. The sensors 788 may comprise a pressure sensor configured to detect a pressure generated by the presence of compressed tissue between the anvil 766 and the staple cartridge 768. The sensors 788 may be configured to detect impedance of a tissue section located between the anvil 766 and the staple cartridge 768 that is indicative of the thickness and/or fullness of tissue located therebetween.

The sensors 788 may be is configured to measure forces exerted on the anvil 766 by a closure drive system. For example, one or more sensors 788 can be at an interaction point between a closure tube and the anvil 766 to detect the closure forces applied by a closure tube to the anvil 766. The forces exerted on the anvil 766 can be representative of the tissue compression experienced by the tissue section captured between the anvil 766 and the staple cartridge 768. The one or more sensors 788 can be positioned at various interaction points along the closure drive system to detect the closure forces applied to the anvil 766 by the closure drive system. The one or more sensors 788 may be sampled in real time during a clamping operation by a processor of the control circuit 760. The control circuit 760 receives real-time sample measurements to provide and analyze time-based information and assess, in real time, closure forces applied to the anvil 766.

A current sensor 786 can be employed to measure the current drawn by the motor 754. The force required to advance the I-beam 764 corresponds to the current drawn by the motor 754. The force is converted to a digital signal and provided to the control circuit 760.

The control circuit 760 can be configured to simulate the response of the actual system of the instrument in the software of the controller. A displacement member can be actuated to move an I-beam 764 in the end effector 752 at or near a target velocity. The surgical instrument 750 can include a feedback controller, which can be one of any feedback controllers, including, but not limited to a PID, a state feedback, LQR, and/or an adaptive controller, for example. The surgical instrument 750 can include a power source to convert the signal from the feedback controller into a physical input such as case voltage, PWM voltage, frequency modulated voltage, current, torque, and/or force, for example.

The actual drive system of the surgical instrument 750 is configured to drive the displacement member, cutting member, or I-beam 764, by a brushed DC motor with gearbox and mechanical links to an articulation and/or knife system. Another example is the electric motor 754 that operates the displacement member and the articulation driver, for example, of an interchangeable shaft assembly. An outside influence is an unmeasured, unpredictable influence of things like tissue, surrounding bodies and friction on the physical system. Such outside influence can be referred to as drag which acts in opposition to the electric motor 754. The outside influence, such as drag, may cause the operation of the physical system to deviate from a desired operation of the physical system.

Various example aspects are directed to a surgical instrument 750 comprising an end effector 752 with motor-driven surgical stapling and cutting implements. For example, a motor 754 may drive a displacement member distally and proximally along a longitudinal axis of the end effector 752. The end effector 752 may comprise a pivotable anvil 766 and, when configured for use, a staple cartridge 768 positioned opposite the anvil 766. A clinician may grasp tissue between the anvil 766 and the staple cartridge 768, as described herein. When ready to use the instrument 750, the clinician may provide a firing signal, for example by depressing a trigger of the instrument 750. In response to the firing signal, the motor 754 may drive the displacement member distally along the longitudinal axis of the end effector 752 from a proximal stroke begin position to a stroke end position distal of the stroke begin position. As the displacement member translates distally, an I-beam 764 with a cutting element positioned at a distal end, may cut the tissue between the staple cartridge 768 and the anvil 766.

In various examples, the surgical instrument 750 may comprise a control circuit 760 programmed to control the distal translation of the displacement member, such as the I-beam 764, for example, based on one or more tissue conditions. The control circuit 760 may be programmed to sense tissue conditions, such as thickness, either directly or indirectly, as described herein. The control circuit 760 may be programmed to select a firing control program based on tissue conditions. A firing control program may describe the distal motion of the displacement member. Different firing control programs may be selected to better treat different tissue conditions. For example, when thicker tissue is present, the control circuit 760 may be programmed to translate the displacement member at a lower velocity and/or with lower power. When thinner tissue is present, the control circuit 760 may be programmed to translate the displacement member at a higher velocity and/or with higher power.

In some examples, the control circuit 760 may initially operate the motor 754 in an open loop configuration for a first open loop portion of a stroke of the displacement member. Based on a response of the instrument 750 during the open loop portion of the stroke, the control circuit 760 may select a firing control program. The response of the instrument may include, a translation distance of the displacement member during the open loop portion, a time elapsed during the open loop portion, energy provided to the motor 754 during the open loop portion, a sum of pulse widths of a motor drive signal, etc. After the open loop portion, the control circuit 760 may implement the selected firing control program for a second portion of the displacement member stroke. For example, during the closed loop portion of the stroke, the control circuit 760 may modulate the motor 754 based on translation data describing a position of the displacement member in a closed loop manner to translate the displacement member at a constant velocity. Additional details are disclosed in U.S. Patent Application Serial No. 15/720,852, titled SYSTEM AND METHODS FOR CONTROLLING A DISPLAY OF A SURGICAL INSTRUMENT, filed September 29, 2017.

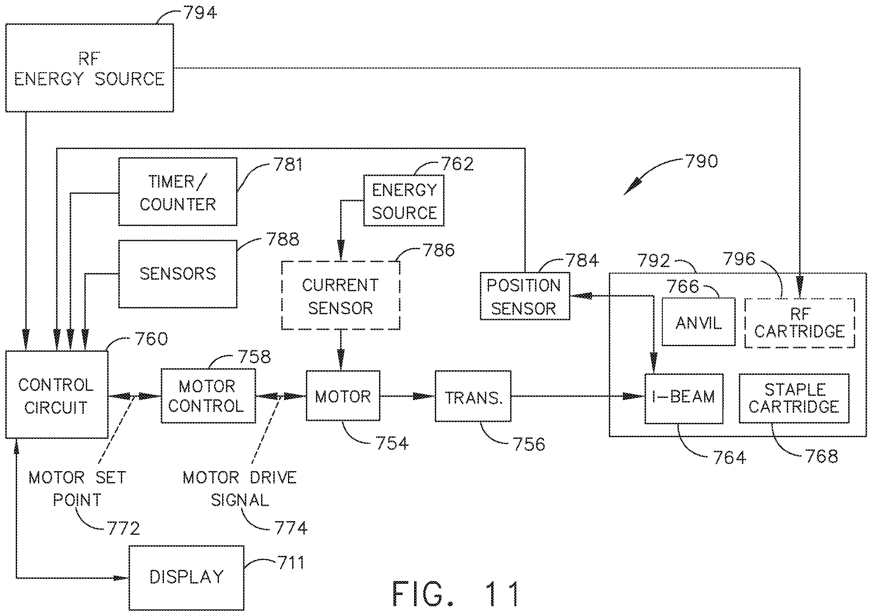

FIG. 11 is a schematic diagram of a surgical instrument 790 configured to control various functions according to one aspect of this disclosure. In one aspect, the surgical instrument 790 is programmed to control distal translation of a displacement member such as the I-beam 764. The surgical instrument 790 comprises an end effector 792 that may comprise an anvil 766, an I-beam 764, and a removable staple cartridge 768 which may be interchanged with an RF cartridge 796 (shown in dashed line).

In one aspect, sensors 788 may be implemented as a limit switch, electromechanical device, solid-state switches, Hall-effect devices, MR devices, GMR devices, magnetometers, among others. In other implementations, the sensors 638 may be solid-state switches that operate under the influence of light, such as optical sensors, IR sensors, ultraviolet sensors, among others. Still, the switches may be solid-state devices such as transistors (e.g., FET, junction FET, MOSFET, bipolar, and the like). In other implementations, the sensors 788 may include electrical conductorless switches, ultrasonic switches, accelerometers, and inertial sensors, among others.

In one aspect, the position sensor 784 may be implemented as an absolute positioning system comprising a magnetic rotary absolute positioning system implemented as an AS5055EQFT single-chip magnetic rotary position sensor available from Austria Microsystems, AG. The position sensor 784 may interface with the control circuit 760 to provide an absolute positioning system. The position may include multiple Hall-effect elements located above a magnet and coupled to a CORDIC processor, also known as the digit-by-digit method and Volder's algorithm, that is provided to implement a simple and efficient algorithm to calculate hyperbolic and trigonometric functions that require only addition, subtraction, bitshift, and table lookup operations.

In one aspect, the I-beam 764 may be implemented as a knife member comprising a knife body that operably supports a tissue cutting blade thereon and may further include anvil engagement tabs or features and channel engagement features or a foot. In one aspect, the staple cartridge 768 may be implemented as a standard (mechanical) surgical fastener cartridge. In one aspect, the RF cartridge 796 may be implemented as an RF cartridge. These and other sensors arrangements are described in commonly owned U.S. Patent Application Serial No. 15/628,175, titled TECHNIQUES FOR ADAPTIVE CONTROL OF MOTOR VELOCITY OF A SURGICAL STAPLING AND CUTTING INSTRUMENT, filed June 20, 2017.

The position, movement, displacement, and/or translation of a linear displacement member, such as the I-beam 764, can be measured by an absolute positioning system, sensor arrangement, and position sensor represented as position sensor 784. Because the I-beam 764 is coupled to the longitudinally movable drive member, the position of the I-beam 764 can be determined by measuring the position of the longitudinally movable drive member employing the position sensor 784. Accordingly, in the following description, the position, displacement, and/or translation of the I-beam 764 can be achieved by the position sensor 784 as described herein. A control circuit 760 may be programmed to control the translation of the displacement member, such as the I-beam 764, as described herein. The control circuit 760, in some examples, may comprise one or more microcontrollers, microprocessors, or other suitable processors for executing instructions that cause the processor or processors to control the displacement member, e.g., the I-beam 764, in the manner described. In one aspect, a timer/counter 781 provides an output signal, such as the elapsed time or a digital count, to the control circuit 760 to correlate the position of the I-beam 764 as determined by the position sensor 784 with the output of the timer/counter 781 such that the control circuit 760 can determine the position of the I-beam 764 at a specific time (t) relative to a starting position. The timer/counter 781 may be configured to measure elapsed time, count external events, or time external events.

The control circuit 760 may generate a motor set point signal 772. The motor set point signal 772 may be provided to a motor controller 758. The motor controller 758 may comprise one or more circuits configured to provide a motor drive signal 774 to the motor 754 to drive the motor 754 as described herein. In some examples, the motor 754 may be a brushed DC electric motor. For example, the velocity of the motor 754 may be proportional to the motor drive signal 774. In some examples, the motor 754 may be a brushless DC electric motor and the motor drive signal 774 may comprise a PWM signal provided to one or more stator windings of the motor 754. Also, in some examples, the motor controller 758 may be omitted, and the control circuit 760 may generate the motor drive signal 774 directly.

The motor 754 may receive power from an energy source 762. The energy source 762 may be or include a battery, a super capacitor, or any other suitable energy source. The motor 754 may be mechanically coupled to the I-beam 764 via a transmission 756. The transmission 756 may include one or more gears or other linkage components to couple the motor 754 to the I-beam 764. A position sensor 784 may sense a position of the I-beam 764. The position sensor 784 may be or include any type of sensor that is capable of generating position data that indicate a position of the I-beam 764. In some examples, the position sensor 784 may include an encoder configured to provide a series of pulses to the control circuit 760 as the I-beam 764 translates distally and proximally. The control circuit 760 may track the pulses to determine the position of the I-beam 764. Other suitable position sensors may be used, including, for example, a proximity sensor. Other types of position sensors may provide other signals indicating motion of the I-beam 764. Also, in some examples, the position sensor 784 may be omitted. Where the motor 754 is a stepper motor, the control circuit 760 may track the position of the I-beam 764 by aggregating the number and direction of steps that the motor has been instructed to execute. The position sensor 784 may be located in the end effector 792 or at any other portion of the instrument.

The control circuit 760 may be in communication with one or more sensors 788. The sensors 788 may be positioned on the end effector 792 and adapted to operate with the surgical instrument 790 to measure the various derived parameters such as gap distance versus time, tissue compression versus time, and anvil strain versus time. The sensors 788 may comprise a magnetic sensor, a magnetic field sensor, a strain gauge, a pressure sensor, a force sensor, an inductive sensor such as an eddy current sensor, a resistive sensor, a capacitive sensor, an optical sensor, and/or any other suitable sensor for measuring one or more parameters of the end effector 792. The sensors 788 may include one or more sensors.

The one or more sensors 788 may comprise a strain gauge, such as a micro-strain gauge, configured to measure the magnitude of the strain in the anvil 766 during a clamped condition. The strain gauge provides an electrical signal whose amplitude varies with the magnitude of the strain. The sensors 788 may comprise a pressure sensor configured to detect a pressure generated by the presence of compressed tissue between the anvil 766 and the staple cartridge 768. The sensors 788 may be configured to detect impedance of a tissue section located between the anvil 766 and the staple cartridge 768 that is indicative of the thickness and/or fullness of tissue located therebetween.

The sensors 788 may be is configured to measure forces exerted on the anvil 766 by the closure drive system. For example, one or more sensors 788 can be at an interaction point between a closure tube and the anvil 766 to detect the closure forces applied by a closure tube to the anvil 766. The forces exerted on the anvil 766 can be representative of the tissue compression experienced by the tissue section captured between the anvil 766 and the staple cartridge 768. The one or more sensors 788 can be positioned at various interaction points along the closure drive system to detect the closure forces applied to the anvil 766 by the closure drive system. The one or more sensors 788 may be sampled in real time during a clamping operation by a processor portion of the control circuit 760. The control circuit 760 receives real-time sample measurements to provide and analyze time-based information and assess, in real time, closure forces applied to the anvil 766.

A current sensor 786 can be employed to measure the current drawn by the motor 754. The force required to advance the I-beam 764 corresponds to the current drawn by the motor 754. The force is converted to a digital signal and provided to the control circuit 760.

An RF energy source 794 is coupled to the end effector 792 and is applied to the RF cartridge 796 when the RF cartridge 796 is loaded in the end effector 792 in place of the staple cartridge 768. The control circuit 760 controls the delivery of the RF energy to the RF cartridge 796.

Additional details are disclosed in U.S. Patent Application Serial No. 15/636,096, titled SURGICAL SYSTEM COUPLABLE WITH STAPLE CARTRIDGE AND RADIO FREQUENCY CARTRIDGE, AND METHOD OF USING SAME, filed June 28, 2017.

Having described a general implementation the of the various surgical instruments, such as surgical instrument 10, and control systems thereof, such as control system 470, the disclosure now turns to describe various other aspects of other surgical instruments. For the sake of brevity, various details of the other surgical instruments and control systems being described in the following sections, which are similar to the various surgical instruments and control systems described above, are not repeated herein. Any aspect of the other surgical instruments and control systems described below can be brought into the above surgical instruments and control systems.

Various surgical instruments described herein may be used to cut and fasten tissue. This may be accomplished by causing a motor of the surgical instrument to drive a firing bar to actuate an end effector. For example, as explained in more detail above, end effector 300 of FIG. 3 includes a firing bar 172 and an I-beam 178. Longitudinally translating firing bar 172 into end effector 300 can cause the actuation, or firing, of staple cartridge 304. The force required to cause this actuation (e.g., firing the surgical instrument) is sometimes referred to herein as a resistive load force, a firing force, or a force-to-fire (FTF).

The data generated by measuring or otherwise determining the resistive load force may be utilized in various ways. For example, based on this force data, an operating parameter of the surgical instrument may be adjusted. Operating parameters that may be adjusted based on force data may include the firing speed or the maximum allowable force of the surgical instrument. Additionally, a target cutline position of the surgical instrument may be adjusted based on force data. Further, the firing force may be determined through multiple means and may be used for redundant force control monitoring. Yet further, as discussed in detail below, firing force data may be logged in a non-volatile storage medium.

In some aspects, the force required to fire the surgical instrument may be measured by a load sensor for sensing the force applied to the firing bar or I-beam. Alternatively, this force may be determined by measuring the current drawn by the motor used to drive the firing bar. For example, as explained in more detail above, sensor 476 of FIG. 4 may be a load sensor that is used to measure the firing force applied to an I-beam during a firing stroke of the surgical instrument or tool. Additionally, current sensor 478 can be employed to measure the current drawn by the motor 482, wherein the motor current draw corresponds to the force required to advance the firing member. However, it may be desirable to determine the force required to fire or actuate the surgical instrument using means that do not involve a direct measurement of the load on the I-beam or the current draw of the motor. Various aspects of determining the force required to fire a surgical instrument are discussed below.

In various aspects, a resistive load encounter by the surgical instrument (i.e, the force required to fire the surgical instrument) may be determined based on a measured voltage of one or more batteries used to power the surgical instrument, an actuator velocity (e.g., a firing member velocity), and, in some cases, a pulse width modulation (PWM) value. Each of the measured battery voltage, actuation velocity, and PWM value may be determined as described below.

With respect to the measured battery voltage, the surgical instrument may include an electric motor powered by a power source that includes one or more batteries. During the firing or actuation of the surgical instrument, the motor is activated and the voltage across the motor power source (e.g., the voltage across the one or more batteries) is measured by a control system associated with the surgical instrument. For example, the control system may be similar to control system 470 shown in FIG. 4, including motor 482, motor driver 492, and microcontroller 461. Additionally, the power source may be similar to power source 90 described above.

With respect to the actuator velocity, the surgical instrument may include an actuator (e.g., a firing member) that, when translated into an end effector by the motor, causes the surgical instrument to fire. The position (e.g., displacement) of the actuator is monitored by a position sensor in communication with a microcontroller. For example, the position sensor and microcontroller may be similar to position sensor 472 and microcontroller 461 shown in FIG. 4, respectively. The microcontroller determines the actuator velocity by comparing the sensed position change of the actuator over time.

With respect to the PWM value, the control system associated with the surgical instrument may be configured to control the power delivered to the motor using pulse width modulation. The pulse width modulation may be controlled by a microcontroller or motor driver similar to microcontroller 461 and motor driver 492 shown in FIG. 4, respectively. The PWM value is determined by the microcontroller based on the PWM percentage used to control power delivery to the motor during firing of the surgical instrument.

In various aspects, an instrument velocity constant and an open loop velocity are used to determine the resistive load encountered when firing the surgical instrument. As used herein, the instrument velocity constant is a constant describing the velocity at which the actuator (e.g. firing member) will travel when a given voltage is applied to the motor (i.e. the motor volt characterization). The instrument velocity constant may be predetermined for a given surgical instrument. The open loop velocity is calculated according to the following relationship:

$$v_{ol} = K_v \; x \; V_m \; x \; PWM$$

In the equation above, $v_{ol}$ is the open loop velocity, $K_v$ is the instrument velocity constant, $V_m$ is the measured battery voltage during actuation of the firing member, and *PWM* is the pulse width modulation value used during actuation of the firing member. For a given firing, the open loop velocity $v_o$ represents the velocity at which the actuator would have traveled if no resistive load was applied to the surgical instrument. The open loop velocity may be calculated by a microcontroller, such as microcontroller 461.

In various aspects, a maximum output force is used to determine the resistive load encountered when firing the surgical instrument. As used herein, the maximum output force is the maximum force the motor is capable of applying at a given power level. Further, the maximum output force may be determined based on the properties of the motor, the measured battery voltage during actuation of the firing member, and/or the PWM value used during actuation of the firing member. In some aspects, for a given firing, a microprocessor determines the maximum output force based on a characterization table or a characterization equation that accounts for the measured battery voltage and, if applicable, the PWM value.

In various aspects, the maximum output force, the open loop velocity, and the actuator velocity during the firing are used to determine the resistive load encountered when firing the surgical instrument. Specifically, the resistive load force may be calculated based on the following relationship:

$$F_r = F_{max} \; x \; \left( \frac{v_{ol} - v_a}{v_{ol}} \right)$$

In the equation above, $F_r$ is the resistive load force, $F_{max}$ is the maximum output force, $v_o$ is the open loop velocity, and $v_a$ is the actuator velocity. This calculation may be performed by a control system of the surgical instrument. Thus, without directly measuring motor current or using load sensors, the resistive load force can be derived based on the battery voltage, PWM value, and actuator velocity during firing of the surgical instrument.

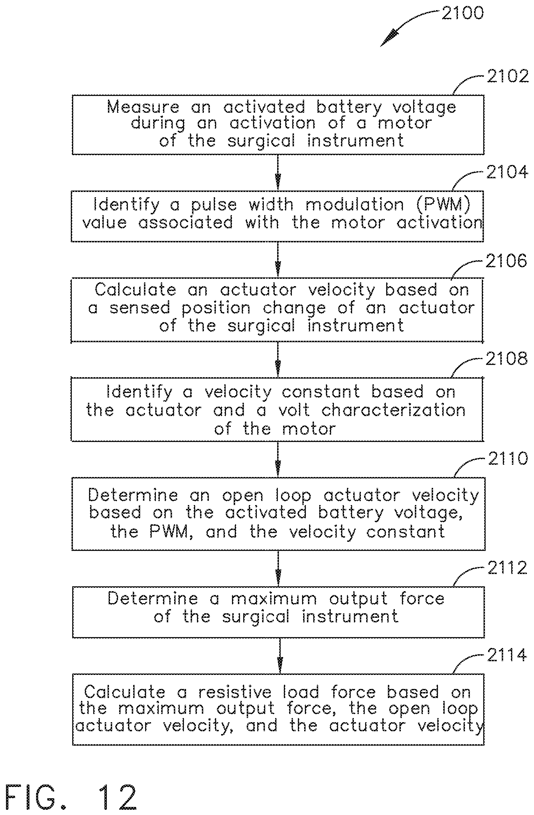

FIG. 12 is a logic flow diagram 2100 of a process depicting a control program or a logic configuration for determining a resistive load force based on battery voltage, velocity, and PWM according to various aspects of this disclosure. In one aspect, a microcontroller, such as microcontroller 416, comprises a processor and a memory coupled to the processor. The memory stores instructions executable by the processor to: 2102 measure an activated battery voltage during an activation of a motor of the surgical instrument; 2104 identify a pulse width modulation (PWM) value associated with the motor activation; 2106 calculate an actuator velocity based on a sensed position change of an actuator of the surgical instrument; 2108 identify a velocity constant based on the actuator and a volt characterization of the motor; 2110 determine an open loop actuator velocity based on the activated battery voltage, the PWM, and the velocity constant; 2112 determine a maximum output force of the surgical instrument; and 2114 calculate a resistive load force based on the maximum output force, the open loop actuator velocity, and the actuator velocity. In other aspects, instructions 2102, 2104, 2106, 2108, 2110, 2112, and 2114 may be stored on a memory and executed by a processor, wherein both the memory and processor are comprised in a surgical hub communicably coupled to the surgical instrument, such as, for example, one of the hubs discussed in U.S. Patent Application Serial No. 15/940,632, entitled DATA STRIPPING METHOD TO INTERROGATE PATIENT RECORDS AND CREATE ANONYMIZED RECORD; Attorney Docket No. END8500USNP/170767.

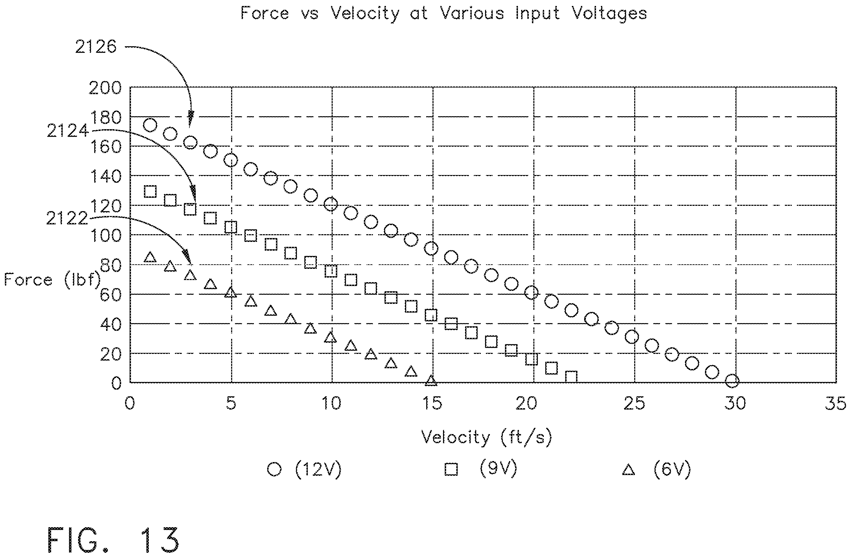

FIG. 13 is a graphical illustration of an approximated resistive load force versus velocity curve at various motor input voltages according to one aspect of this disclosure. Specifically, FIG. 13 illustrates the inverse relationship between resistive load force and actuator firing velocity when various input voltages are applied to power the drive motor. For example, as shown by data points 2122, the calculated resistive load forces decrease as actuator firing velocity increase when a voltage of 6V, is applied to the drive motor. A similar relationship is shown by data points 2124 and 2126 at input voltages of 9V and 12V, respectively. Thus, using this relationship, measuring the voltage across the motor power supply (i.e. the battery voltage) and determining an actuator velocity based on position sensor data allows the resistive load force encountered during the firing of the surgical instrument to be approximated.

As explained in detail above, data generated by the surgical instrument control system related to the resistive load force may be utilized in various ways. For example, based on this data, an operating parameter of the surgical instrument may be adjusted. Additionally, this data may be logged in a non-volatile storage medium for future access or used for redundant force control monitoring.

In various aspects, a resistive load encountered by the surgical instrument (i.e. the force required to fire the surgical instrument) may be determined based on an activated voltage across one or more batteries during firing, a baseline voltage across the one or more batteries when the instrument is not firing, and actuator velocity (e.g., a firing member velocity). Each of the activated battery voltage, the baseline voltage, and the actuator velocity may be determined as described below.

With respect to the activated battery voltage and the baseline battery voltage, the surgical instrument may include an electric motor powered by a power source that includes one or more batteries. During the firing or actuation of the surgical instrument, the motor is activated and the voltage across the motor power source (e.g., the voltage across the one or more batteries) is measured by a control system associated with the surgical instrument. This measured voltage is the activated battery voltage. Similarly, the voltage across the power source may be measured when the surgical instrument is under a low load condition. This measured voltage is the baseline battery voltage. A low load condition may exist, for example, when the surgical instrument is powered on but is not being fired. Further, the control system associated with the surgical instrument may be similar to control system 470 shown in FIG. 4, including motor 482, motor driver 492, and microcontroller 461. Additionally, the power source may be similar to power source 90 described above.

With respect to the actuator velocity, the surgical instrument may include an actuator (e.g., a firing member) that, when translated into an end effector by the motor, causes the surgical instrument to fire. The position (e.g., displacement) of the actuator is monitored by a position sensor in communication with a microcontroller. For example, the position sensor and microcontroller may be similar to position sensor 472 and microcontroller 461 shown in FIG. 4, respectively. The microcontroller determines the actuator velocity by comparing the sensed position change of the actuator over time.

In various aspects, a battery voltage decrease and a battery output power may be calculated to determine the resistive load encountered when firing the surgical instrument. As used herein, the battery voltage decrease is the difference between the activated battery voltage during firing and the baseline battery voltage. Based on this battery voltage decrease, the battery output power may be approximated (i.e. the amount of power output by the battery during the firing of the surgical instrument).

In various aspects, the battery output power and the actuator velocity during the firing, and in some cases, an efficiency factor, are used to determine the resistive load encountered when firing the surgical instrument. Specifically, the resistive load force may be calculated based on the following relationship:

$$F_r = c_{eff} \left( \frac{P_{battery}}{v_a} \right)$$

In the equation above, $F_r$ is the resistive load force, $P_{battery}$ is the battery output power, $v_a$ is the actuator velocity, and $c_{eff}$ is an efficiency factor. In some aspects, the efficiency factor may depend on the actuator velocity. For example, $c_{eff}$ may be determined based on a characterization table or a characterization equation that is dependent upon $v_a$. In other aspects, the efficiency factor may be a constant that is predetermined for a given surgical instrument. Thus, using the equation above, and without directly measuring motor current or using load sensors, the resistive load force may be approximated based on a baseline voltage across one or more batteries of a surgical instrument when the instrument is not firing, an activated voltage across the one or more batteries during firing, and an actuator velocity during firing.

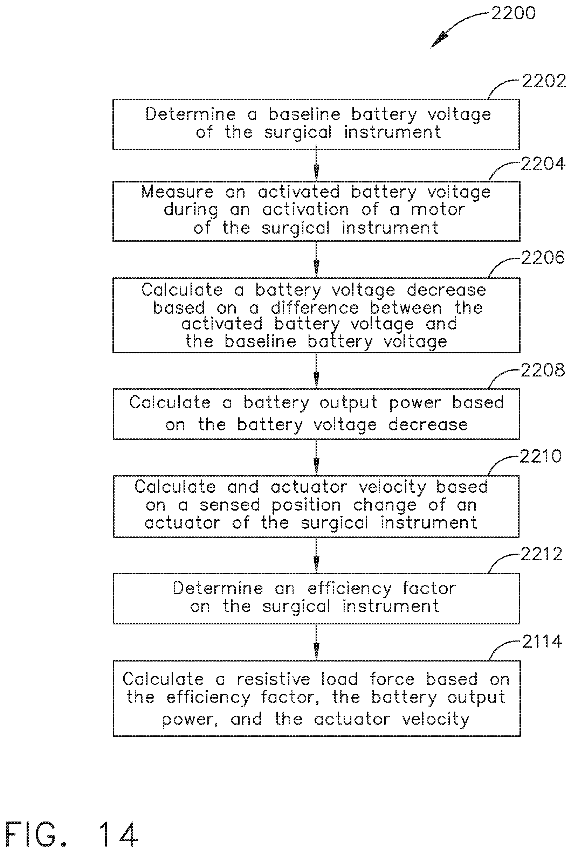

FIG. 14 is a logic flow diagram 2200 of a process depicting a control program or a logic configuration for determining a resistive load force based on a baseline battery voltage, an activated battery voltage, and actuator velocity according to various aspects of this disclosure. In one aspect, a microcontroller, such as microcontroller 416, comprises a processor and a memory coupled to the processor. The memory stores instructions executable by the processor to: 2202 determine a baseline battery voltage of the surgical instrument; 2204 measure an activated battery voltage during an activation of a motor of the surgical instrument; 2206 calculate a battery voltage decrease based on a difference between the activated battery voltage and the baseline battery voltage; 2208 calculate a battery output power based on the battery voltage decrease; 2210 calculate an actuator velocity based on a sensed position change of an actuator of the surgical instrument; 2212 determine an efficiency factor of the surgical instrument; and 2214 calculate a resistive load force based on the efficiency factor, the battery output power, and the actuator velocity. In other aspects, instructions 2202, 2204, 2206, 2208, 2210, 2212, and 2214 may be stored on a memory and executed by a processor, wherein both the memory and processor are comprised in a surgical hub communicably coupled to the surgical instrument, such surgical, for example one of the hubs discussed in the aforementioned reference, U.S. Patent Application Serial No. 15/940,632, entitled DATA STRIPPING METHOD TO INTERROGATE PATIENT RECORDS AND CREATE ANONYMIZED RECORD; Attorney Docket No. END8500USNP/170767.

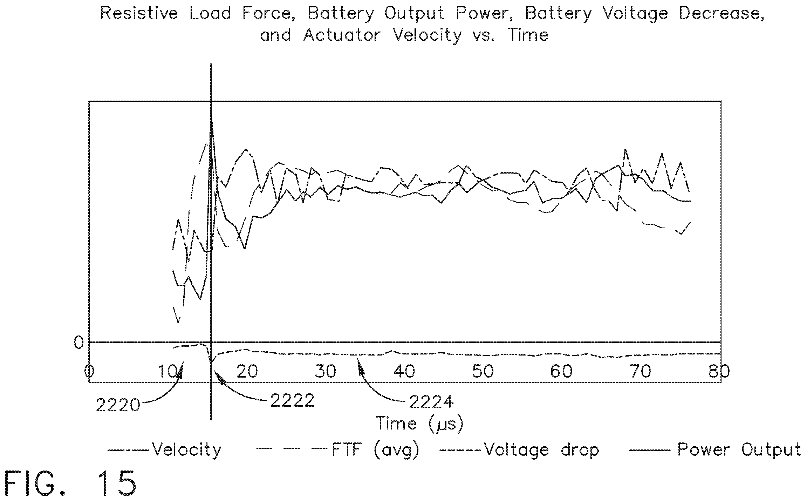

FIG. 15 is a graphical illustration of a resistive load force, battery output power, battery voltage decrease, and actuator velocity versus time curve for an exemplary firing of a surgical instrument according to one aspect of this disclosure. Specifically, FIG. 15 illustrates an average resistive load force calculated based on a baseline voltage, an activated voltage, and an actuator velocity, as described in various aspects above. At time point 2220, the voltage drop is approximately zero, representing a battery voltage near the baseline voltage. At time point 2222, a sudden decrease in the battery voltage drop is observed as the instrument is firing. As the battery voltage drop decreases, the calculated battery output power and average resistive load force (FTF) shows a corresponding increase. After the battery output power and average resistive load force spike, these values show a relative leveling out at time point 2224.

As explained in detail above, data generated by the surgical instrument control system related to the resistive load force may be utilized in various ways. For example, based on this data, an operating parameter of the surgical instrument may be adjusted. Additionally, this data may be logged in a non-volatile storage medium for future access or used for redundant force control monitoring.

As discussed in detail above, during the operation of the various surgical instruments discussed herein, instrument operating parameters may be measured, calculated, or otherwise determined. For example, instrument operating parameters may be determined by a control system (e.g., control system 470 of FIG. 4) during an actuation or firing of the surgical instrument.

It may be useful to access data related to measured, calculated, or otherwise determined instrument operating parameters following a use of the surgical instrument (e.g., after the instrument has been actuated or fired). For example, it may be useful to analyze operating parameter data corresponding to the firing of a surgical instrument as part of a clinical study related to the instrument. In other cases, in may be useful to analyze operating parameter data corresponding to the firing of a surgical instrument as part of design and verification testing for the instrument. In yet other cases, it may be useful to access and present this operating parameter data as part of a device product inquiry analysis. Accordingly, there is a need for devices, systems, and methods for logging and storing data related to various operating parameters that are measured, calculated, or otherwise determined during the operation of surgical instruments.

In various aspects, instrument operating parameter data associated with the actuation or firing of a surgical instrument may be stored in a non-volatile storage medium so that it may be retrieved following a use of the instrument. For example, during a firing of a surgical instrument, operating parameter data may be generated by a control system of the surgical instrument (e.g., control system 470 of FIG. 4). After the firing, the control system may cause the generated data to be written to a non-volatile storage medium. In some aspects, the non-volatile storage medium may be located local to the surgical instrument (e.g., memory 448 of FIG. 4 may be a non-volatile storage medium). In other aspects, the non-volatile storage medium may be located in a surgical hub communicably coupled to the control system of surgical instrument, for example, one of the surgical hubs discussed in the aforementioned reference, U.S. Patent Application Serial No. 15/940,632, entitled DATA STRIPPING METHOD TO INTERROGATE PATIENT RECORDS AND CREATE ANONYMIZED RECORD; Attorney Docket No. END8500USNP/170767. As used herein, a non-volatile storage medium refers to any type of storage medium that is configured to store data even when power is removed from the storage device.

In other aspects, the surgical instrument control system may cause the generated data to be written to a non-volatile storage medium upon an event other than the successful firing of the instrument. For example, there may be instances where a stall event occurs when attempting to fire the surgical instrument. As used herein, a stall event may be described as an event that prevents the surgical instrument from completely firing. Such a stall event may occur if something causes the instrument motor to stop (e.g., something obstructs the end effector of the surgical instrument), preventing the instrument from completing an actuation. As the result of a stall event, a processor of the instrument control system, such as processor 462 of FIG. 4, may need to devote additional processing power to motion control of the instrument. Accordingly, the control system may cause operating parameter data generated during the attempted firing of the instrument to be written to the non-volatile storage medium after a stall event has accrued. This may minimize processing resources allocated to writing operating data in order to ensure that there is minimal interference with the motion control processing needs of the control system.

A control system of the surgical instrument may cause many different types of operating parameter data to be generated during the firing of a surgical instrument and to be stored in a non-volatile storage medium. For example, a control system of the surgical instrument may cause timestamp data that is related to the firing of the instrument to be generated and stored. In some aspects, this timestamp data may be based on microseconds of device operation relative to a reference time.

Position data related the firing of a surgical instrument may be generated and stored to a non-volatile storage medium. For example, as discussed in detail above, position (e.g., displacement of the actuator compared to a reference point) data may be generated by a position sensor in communication with a microcontroller. For example, the position sensor and microcontroller may be similar to position sensor 472 and microcontroller 461 shown in FIG. 4, respectively.

Velocity data related the firing of a surgical instrument may be generated and stored to a non-volatile storage medium. For example, as discussed in detail above, the control system may generate data related to actuator velocity by comparing the sensed position change of the actuator over time (i.e. the position change of the actuator is monitored by a position sensor in communication with a microcontroller).

Pulse Width Modulation (PWM) data related the firing of a surgical instrument may be generated and stored to a non-volatile storage medium. For example, as discussed in detail above, the control system may select a PWM value to control the power delivered to the motor using pulse width modulation. Further, in some aspects, this PMW value may be determined by the control system based on an error in the calculation of the velocity data.

Battery voltage data related the firing of a surgical instrument may be generated and stored to a non-volatile storage medium. For example, as discussed in detail above, the surgical instrument may include an electric motor powered by a power source that includes one or more batteries. During the firing or actuation of the surgical instrument, the motor is activated and the voltage across the motor power source (e.g., the voltage across the one or more batteries) may be measured by the control system associated with the surgical instrument.

Force data related the firing of a surgical instrument may be generated and stored to a non-volatile storage medium. As discussed in detail above, there may be numerous ways to generate force data. For example, the force required to fire the surgical instrument may be measured by a load sensor for sensing the force applied to a firing bar or I-beam. Alternatively, this force may be determined by measuring the current drawn by the motor used to drive the firing bar. In yet other aspects, force data may be generated using alternate means such as calculating force based on battery voltage, velocity, and PWM values or based on velocity and battery voltage decrease.

Moreover, in various aspects, the rate at which the various operating parameter data is generated and captured may be determined by the control system using a parameterized value. This parameterized value may be adjusted and optimized based on the data generation and data analysis needs.

In various aspects, a control program or a logic configuration for a method of storing data associated with a firing of a surgical stapling instrument to a non-volatile computer readable storage medium is disclosed. In one aspect, a microcontroller, such as microcontroller 416, comprises a processor and a memory coupled to the processor. The memory stores instructions executable by the processor to: capture an activation timestamp during activation of a motor of the surgical instrument; measure a position of an actuator of the surgical instrument during the activation; calculate an actuator velocity based on a sensed position change of the actuator during the activation; identify a pulse width modulation (PWM) value associated with the activation; measure an activated battery voltage the activation; calculate a resistive load force encountered during the activation; and write data associated with any of the activation timestamp, actuator position, actuator velocity, PWM value, activated battery voltage, and resistive load force to the non-volatile computer readable storage medium.

While several forms have been illustrated and described, it is not the intention of Applicant to restrict or limit the scope of the appended claims to such detail. Numerous modifications, variations, changes, substitutions, and combinations to those forms may be implemented and will occur to those skilled in the art without departing from the scope of the present disclosure. Moreover, the structure of each element associated with the described forms can be alternatively described as a means for providing the function performed by the element. Also, where materials are disclosed for certain components, other materials may be used. It is therefore to be understood that the foregoing description and the appended claims are intended to cover all such modifications, combinations, and variations as falling within the scope of the disclosed forms.

The foregoing detailed description has set forth various forms of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, and/or examples can be implemented, individually and/or collectively, by a wide range of hardware, software, firmware, or virtually any combination thereof. Those skilled in the art will recognize that some aspects of the forms disclosed herein, in whole or in part, can be equivalently implemented in integrated circuits, as one or more computer programs running on one or more computers (e.g., as one or more programs running on one or more computer systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure. In addition, those skilled in the art will appreciate that the mechanisms of the subject matter described herein are capable of being distributed as one or more program products in a variety of forms, and that an illustrative form of the subject matter described herein applies regardless of the particular type of signal bearing medium used to actually carry out the distribution.

Instructions used to program logic to perform various disclosed aspects can be stored within a memory in the system, such as dynamic random access memory (DRAM), cache, flash memory, or other storage. Furthermore, the instructions can be distributed via a network or by way of other computer readable media. Thus a machine-readable medium may include any mechanism for storing or transmitting information in a form readable by a machine (e.g., a computer), but is not limited to, floppy diskettes, optical disks, compact disc, read-only memory (CD-ROMs), and magneto-optical disks, read-only memory (ROMs), random access memory (RAM), erasable programmable read-only memory (EPROM), electrically erasable programmable read-only memory (EEPROM), magnetic or optical cards, flash memory, or a tangible, machine-readable storage used in the transmission of information over the Internet via electrical, optical, acoustical or other forms of propagated signals (e.g., carrier waves, infrared signals, digital signals, etc.). Accordingly, the non-transitory computer-readable medium includes any type of tangible machine-readable medium suitable for storing or transmitting electronic instructions or information in a form readable by a machine (e.g., a computer).

As used in any aspect herein, the term "control circuit" may refer to, for example, hardwired circuitry, programmable circuitry (e.g., a computer processor including one or more individual instruction processing cores, processing unit, processor, microcontroller, microcontroller unit, controller, digital signal processor (DSP), programmable logic device (PLD), programmable logic array (PLA), or field programmable gate array (FPGA)), state machine circuitry, firmware that stores instructions executed by programmable circuitry, and any combination thereof. The control circuit may, collectively or individually, be embodied as circuitry that forms part of a larger system, for example, an integrated circuit (IC), an application-specific integrated circuit (ASIC), a system on-chip (SoC), desktop computers, laptop computers, tablet computers, servers, smart phones, etc. Accordingly, as used herein "control circuit" includes, but is not limited to, electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of random access memory), and/or electrical circuitry forming a communications device (e.g., a modem, communications switch, or optical-electrical equipment). Those having skill in the art will recognize that the subject matter described herein may be implemented in an analog or digital fashion or some combination thereof.

As used in any aspect herein, the term "logic" may refer to an app, software, firmware and/or circuitry configured to perform any of the aforementioned operations. Software may be embodied as a software package, code, instructions, instruction sets and/or data recorded on non-transitory computer readable storage medium. Firmware may be embodied as code, instructions or instruction sets and/or data that are hard-coded (e.g., nonvolatile) in memory devices.

As used in any aspect herein, the terms "component," "system," "module" and the like can refer to a computer-related entity, either hardware, a combination of hardware and software, software, or software in execution.

As used in any aspect herein, an "algorithm" refers to a self-consistent sequence of steps leading to a desired result, where a "step" refers to a manipulation of physical quantities and/or logic states which may, though need not necessarily, take the form of electrical or magnetic signals capable of being stored, transferred, combined, compared, and otherwise manipulated. It is common usage to refer to these signals as bits, values, elements, symbols, characters, terms, numbers, or the like. These and similar terms may be associated with the appropriate physical quantities and are merely convenient labels applied to these quantities and/or states.

A network may include a packet switched network. The communication devices may be capable of communicating with each other using a selected packet switched network communications protocol. One example communications protocol may include an Ethernet communications protocol which may be capable permitting communication using a Transmission Control Protocol/Internet Protocol (TCP/IP). The Ethernet protocol may comply or be compatible with the Ethernet standard published by the Institute of Electrical and Electronics Engineers (IEEE) titled "IEEE 802.3 Standard", published in December, 2008 and/or later versions of this standard. Alternatively or additionally, the communication devices may be capable of communicating with each other using an X.25 communications protocol. The X.25 communications protocol may comply or be compatible with a standard promulgated by the International Telecommunication Union-Telecommunication Standardization Sector (ITU-T). Alternatively or additionally, the communication devices may be capable of communicating with each other using a frame relay communications protocol. The frame relay communications protocol may comply or be compatible with a standard promulgated by Consultative Committee for International Telegraph and Telephone (CCITT) and/or the American National Standards Institute (ANSI). Alternatively or additionally, the transceivers may be capable of communicating with each other using an Asynchronous Transfer Mode (ATM) communications protocol. The ATM communications protocol may comply or be compatible with an ATM standard published by the ATM Forum titled "ATM-MPLS Network Interworking 2.0" published August 2001, and/or later versions of this standard. Of course, different and/or after-developed connection-oriented network communication protocols are equally contemplated herein.

Unless specifically stated otherwise as apparent from the foregoing disclosure, it is appreciated that, throughout the foregoing disclosure, discussions using terms such as "processing," "computing," "calculating," "determining," "displaying," or the like, refer to the action and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

One or more components may be referred to herein as "configured to," "configurable to," "operable/operative to," "adapted/adaptable," "able to," "conformable/conformed to," etc. Those skilled in the art will recognize that "configured to" can generally encompass active-state components and/or inactive-state components and/or standby-state components, unless context requires otherwise.

The terms "proximal" and "distal" are used herein with reference to a clinician manipulating the handle portion of the surgical instrument. The term "proximal" refers to the portion closest to the clinician and the term "distal" refers to the portion located away from the clinician. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

Those skilled in the art will recognize that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to claims containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations.

In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that typically a disjunctive word and/or phrase presenting two or more alternative terms, whether in the description, claims, or drawings, should be understood to contemplate the possibilities of including one of the terms, either of the terms, or both terms unless context dictates otherwise. For example, the phrase "A or B" will be typically understood to include the possibilities of "A" or "B" or "A and B."

With respect to the appended claims, those skilled in the art will appreciate that recited operations therein may generally be performed in any order. Also, although various operational flow diagrams are presented in a sequence(s), it should be understood that the various operations may be performed in other orders than those which are illustrated, or may be performed concurrently. Examples of such alternate orderings may include overlapping, interleaved, interrupted, reordered, incremental, preparatory, supplemental, simultaneous, reverse, or other variant orderings, unless context dictates otherwise. Furthermore, terms like "responsive to," "related to," or other past-tense adjectives are generally not intended to exclude such variants, unless context dictates otherwise.

It is worthy to note that any reference to "one aspect," "an aspect," "an exemplification," "one exemplification," and the like means that a particular feature, structure, or characteristic described in connection with the aspect is included in at least one aspect. Thus, appearances of the phrases "in one aspect," "in an aspect," "in an exemplification," and "in one exemplification" in various places throughout the specification are not necessarily all referring to the same aspect. Furthermore, the particular features, structures or characteristics may be combined in any suitable manner in one or more aspects.

In summary, numerous benefits have been described which result from employing the concepts described herein. The foregoing description of the one or more forms has been presented for purposes of illustration and description. It is not intended to be exhaustive or limiting to the precise form disclosed. Modifications or variations are possible in light of the above teachings. The one or more forms were chosen and described in order to illustrate principles and practical application to thereby enable one of ordinary skill in the art to utilize the various forms and with various modifications as are suited to the particular use contemplated. It is intended that the claims submitted herewith define the overall scope.

**Claims**

1. A surgical instrument (10, 600, 700, 750, 790), comprising:

   a battery (98, 628, 712, 762);
   a motor (82, 602, 704a, 754) powered by the battery;

   an end effector (300) configured to grasp tissue;
   an actuator coupled to the end effector, wherein the motor is configured to cause the actuator to move to yield a surgical treatment of the tissue by the end effector; and
   **characterised by** a control circuit (620, 710, 760), configured to:

   measure an activated battery voltage during an activation of the motor to move the actuator;
   identify a pulse width modulation (PWM) value associated with the motor activation;
   calculate an actuator velocity based on a sensed position change of the actuator; and
   calculate a resistive load force based on the activated battery voltage, the PWM value, and the actuator velocity.

2. The surgical instrument of claim 1, wherein the control circuit is further configured to:

   identify a velocity constant based on the actuator and a volt characterization of the motor; and
   determine an open loop actuator velocity based on the activated battery voltage, the PWM, and the velocity constant.

3. The surgical instrument of claim 1 or claim 2, wherein the control circuit is further configured to determine a maximum output force of the motor, and, optionally, wherein the control circuit is further configured to calculate the resistive load force based on the open loop actuator velocity and the maximum output force.

4. A surgical instrument (10, 600, 700, 750, 790), comprising:

   a battery (98, 628, 712, 762);
   a motor powered by the battery (82, 602, 704a, 754);
   an end effector (300) configured to grasp tissue;
   an actuator coupled to the end effector, wherein the motor is configured to cause the actuator to move to yield a surgical treatment of the tissue by the end effector;
   **characterised by** a control circuit (620, 710, 760), configured to:

   determine a baseline battery voltage;
   measure an activated battery voltage during an activation of the motor;
   calculate a velocity of the actuator based on a sensed position change of the actuator; and
   calculate a resistive load force based on the baseline battery voltage, the activated battery voltage, and the actuator velocity.

**5.** The surgical instrument of claim 4, wherein the control circuit is further configured to calculate a battery voltage decrease based on a difference between the activated battery voltage and the baseline battery voltage.

**6.** The surgical instrument of claim 5, wherein the control circuit is further configured to calculate a battery output power based on the battery voltage decrease, and, optionally, wherein the control circuit is further configured to calculate the resistive load force based on the battery output power.

**7.** The surgical instrument of any one of claims 4 - 6, wherein the control circuit is further configured to determine an efficiency factor of the surgical instrument, and wherein the resistive load force is calculated based on the efficiency factor, and, optionally, wherein the efficiency factor is dependent upon the actuator velocity.

**8.** The surgical instrument of any preceding claim, wherein the control circuit is further configured to store the calculated resistive load force.

**9.** The surgical instrument of any preceding claim, wherein the control circuit is configured to optimize a target firing position of the surgical instrument based on the calculated resistive load force.

**11.** A computer program product comprising instructions that either:

> when executed by the control circuit of the surgical instrument of claim 1, cause the control circuit (620, 710, 760) to:

>> measure an activated battery voltage during an activation of the motor to move the actuator;
>> identify a pulse width modulation (PWM) value associated with the motor activation;
>> calculate an actuator velocity based on a sensed position change of the actuator; and
>> calculate the resistive load force based on the activated battery voltage, the PWM value, and the actuator velocity; or

> when executed by the control circuit (620, 710, 760) of the surgical instrument of claim 4, cause the control circuit to:

>> determine a baseline battery voltage;
>> measure an activated battery voltage during an activation of the motor;
>> calculate a velocity of the actuator based on a sensed position change of the actuator; and

>> calculate a resistive load force based on the baseline battery voltage, the activated battery voltage, and the actuator velocity.

**12.** A computer-readable medium having stored thereon, or a signal carrying, the computer program product of claim 11.

**Patentansprüche**

**1.** Chirurgisches Instrument (10, 600, 700, 750, 790), umfassend:

> eine Batterie (98, 628, 712, 762);
> einen Motor (82, 602, 704a, 754), der durch die Batterie angetrieben wird;
> einen Endeffektor (300), der konfiguriert ist, um Gewebe zu greifen;
> einen Aktuator, der mit dem Endeffektor gekoppelt ist, wobei der Motor konfiguriert ist, um den Aktuator zu veranlassen, sich zu bewegen, um eine chirurgische Behandlung des Gewebes durch den Endeffektor zu erbringen; und
> **gekennzeichnet durch** eine Steuerschaltung (620, 710, 760), die konfiguriert ist zum:

>> Messen einer aktivierten Batteriespannung während einer Aktivierung des Motors, um den Aktuator zu bewegen;
>> Identifizieren eines Pulsweitenmodulationswerts (PWM-Wert), der der Motoraktivierung zugeordnet ist;
>> Berechnen einer Aktuatorgeschwindigkeit basierend auf einer erfassten Positionsänderung des Aktuators; und
>> Berechnen einer Widerstandsbelastungskraft basierend auf der aktivierten Batteriespannung, dem PWM-Wert und der Aktuatorgeschwindigkeit.

**2.** Chirurgisches Instrument nach Anspruch 1, wobei die Steuerschaltung ferner konfiguriert ist zum: Identifizieren einer Geschwindigkeitskonstante basierend auf dem Aktuator und einer Voltcharakterisierung des Motors; und Bestimmen einer Aktuatorgeschwindigkeit bei offenem Regelkreis basierend auf der aktivierten Batteriespannung, dem PWM und der Geschwindigkeitskonstante.

**3.** Chirurgisches Instrument nach Anspruch 1 oder 2, wobei die Steuerschaltung ferner konfiguriert ist, um eine maximale Ausgangskraft des Motors zu bestimmen, und wobei die Steuerschaltung optional ferner konfiguriert ist, um die Widerstandsbelastungskraft basierend auf der Aktuatorgeschwindigkeit bei offenem Regelkreis und der maximalen Ausgangskraft zu berechnen.

4. Chirurgisches Instrument (10, 600, 700, 750, 790), umfassend:

   eine Batterie (98, 628, 712, 762);
   einen Motor, der durch die Batterie (82, 602, 704a, 754) angetrieben wird;
   einen Endeffektor (300), der konfiguriert ist, um Gewebe zu greifen;
   einen Aktuator, der mit dem Endeffektor gekoppelt ist, wobei der Motor konfiguriert ist, um den Aktuator zu veranlassen, sich zu bewegen, um eine chirurgische Behandlung des Gewebes durch den Endeffektor zu erbringen;
   **gekennzeichnet durch** eine Steuerschaltung (620, 710, 760), die konfiguriert ist zum:

   Bestimmen einer Basisbatteriespannung;
   Messen einer aktivierten Batteriespannung während einer Aktivierung des Motors;
   Berechnen einer Geschwindigkeit des Aktuators basierend auf einer erfassten Positionsänderung des Aktuators; und
   Berechnen einer Widerstandsbelastungskraft basierend auf der Basisbatteriespannung, der aktivierten Batteriespannung und der Aktuatorgeschwindigkeit.

5. Chirurgisches Instrument nach Anspruch 4, wobei die Steuerschaltung ferner konfiguriert ist, um einen Batteriespannungsabfall basierend auf einer Differenz zwischen der aktivierten Batteriespannung und der Basisbatteriespannung zu berechnen.

6. Chirurgisches Instrument nach Anspruch 5, wobei die Steuerschaltung ferner konfiguriert ist, um eine Batterieausgangsleistung basierend auf dem Batteriespannungsabfall zu berechnen, und wobei die Steuerschaltung optional ferner konfiguriert ist, um die Widerstandsbelastungskraft basierend auf der Batterieausgangsleistung zu berechnen.

7. Chirurgisches Instrument nach einem der Ansprüche 4 bis 6, wobei die Steuerschaltung ferner konfiguriert ist, um einen Effizienzfaktor des chirurgischen Instruments zu bestimmen, und wobei die Widerstandsbelastungskraft basierend auf dem Effizienzfaktor berechnet wird und wobei der Effizienzfaktor optional abhängig von der Aktuatorgeschwindigkeit ist.

8. Chirurgisches Instrument nach einem der vorstehenden Ansprüche, wobei die Steuerschaltung ferner konfiguriert ist, um die berechnete Widerstandsbelastungskraft zu speichern.

9. Chirurgisches Instrument nach einem der vorstehenden Ansprüche, wobei die Steuerschaltung konfiguriert ist, um eine Zielauslöseposition des chirurgischen Instruments basierend auf der berechneten Widerstandsbelastungskraft zu optimieren.

11. Computerprogrammprodukt, umfassend Anweisungen, die entweder:
   wenn sie durch die Steuerschaltung des chirurgischen Instruments nach Anspruch 1 ausgeführt werden, die Steuerschaltung (620, 710, 760) veranlassen zum:

   Messen einer aktivierten Batteriespannung während einer Aktivierung des Motors, um den Aktuator zu bewegen;
   Identifizieren eines Pulsweitenmodulationswerts (PWM-Wert), der der Motoraktivierung zugeordnet ist;
   Berechnen einer Aktuatorgeschwindigkeit basierend auf einer erfassten Positionsänderung des Aktuators; und
   Berechnen der Widerstandsbelastungskraft basierend auf der aktivierten Batteriespannung, dem PWM-Wert und der Aktuatorgeschwindigkeit; oder
   wenn sie durch die Steuerschaltung (620, 710, 760) des chirurgischen Instruments nach Anspruch 4 ausgeführt werden, die Steuerschaltung veranlassen zum:

   Bestimmen einer Basisbatteriespannung;
   Messen einer aktivierten Batteriespannung während einer Aktivierung des Motors;
   Berechnen einer Geschwindigkeit des Aktuators basierend auf einer erfassten Positionsänderung des Aktuators; und
   Berechnen einer Widerstandsbelastungskraft basierend auf der Basisbatteriespannung, der aktivierten Batteriespannung und der Aktuatorgeschwindigkeit.

12. Computerlesbares Medium, das das Computerprogrammprodukt nach Anspruch 11 darauf gespeichert aufweist, oder Signal, das dieses trägt.

**Revendications**

1. Instrument chirurgical (10, 600, 700, 750, 790), comprenant :

   une batterie (98, 628, 712, 762) ;
   un moteur (82, 602, 704a, 754) alimenté en puissance par la batterie ;
   un effecteur terminal (300) conçu pour saisir un tissu ;
   un actionneur accouplé à l'effecteur terminal, dans lequel le moteur est conçu pour amener l'actionneur à se déplacer pour produire un traitement chirurgical du tissu par l'effecteur terminal ; et

**caractérisé par** un circuit de commande (620, 710, 760), configuré pour :

mesurer une tension de batterie activée pendant une activation du moteur pour déplacer l'actionneur ;

identifier une valeur de modulation de largeur d'impulsion (PWM) associée à l'activation de moteur ;

calculer une vitesse d'actionneur en fonction d'un changement de position capté de l'actionneur ; et

calculer une force de charge résistive en fonction de la tension de batterie activée, de la valeur PWM et de la vitesse d'actionneur.

2. Instrument chirurgical selon la revendication 1, dans lequel le circuit de commande est configuré en outre pour :

identifier une constante de vitesse en fonction de l'actionneur et d'une caractérisation de volt du moteur ; et

déterminer une vitesse d'actionneur en boucle ouverte en fonction de la tension de batterie activée, de la PWM et de la constante de vitesse.

3. Instrument chirurgical selon la revendication 1 ou la revendication 2, dans lequel le circuit de commande est configuré en outre pour déterminer une force de sortie maximale du moteur et, facultativement, dans lequel le circuit de commande est configuré en outre pour calculer la force de charge résistive en fonction de la vitesse d'actionneur en boucle ouverte et de la force de sortie maximale.

4. Instrument chirurgical (10, 600, 700, 750, 790), comprenant :

une batterie (98, 628, 712, 762) ;

un moteur alimenté en puissance par la batterie (82, 602, 704a, 754) ;

un effecteur terminal (300) conçu pour saisir un tissu ;

un actionneur accouplé à l'effecteur terminal, dans lequel le moteur est conçu pour amener l'actionneur à se déplacer pour produire un traitement chirurgical du tissu par l'effecteur terminal ;

**caractérisé par** un circuit de commande (620, 710, 760), configuré pour :

déterminer une tension de batterie de ligne de base ;

mesurer une tension de batterie activée pendant une activation du moteur ;

calculer une vitesse de l'actionneur en fonction d'un changement de position capté de l'actionneur ; et

calculer une force de charge résistive en fonction de la tension de batterie de ligne de base, de la tension de batterie activée et de la vitesse d'actionneur.

5. Instrument chirurgical selon la revendication 4, dans lequel le circuit de commande est configuré en outre pour calculer une diminution de tension de batterie en fonction d'une différence entre la tension de batterie activée et la tension de batterie de ligne de base.

6. Instrument chirurgical selon la revendication 5, dans lequel le circuit de commande est configuré en outre pour calculer une puissance de sortie de batterie en fonction de la diminution de tension de batterie et, facultativement, dans lequel le circuit de commande est configuré en outre pour calculer la force de charge résistive en fonction de la puissance de sortie de batterie.

7. Instrument chirurgical selon l'une quelconque des revendications 4 à 6, dans lequel le circuit de commande est configuré en outre pour déterminer un facteur d'efficacité de l'instrument chirurgical, et dans lequel la force de charge résistive est calculée en fonction du facteur d'efficacité et, facultativement, dans lequel le facteur d'efficacité dépend de la vitesse d'actionneur.

8. Instrument chirurgical selon l'une quelconque revendication précédente, dans lequel le circuit de commande est configuré en outre pour stocker la force de charge résistive calculée.

9. Instrument chirurgical selon l'une quelconque revendication précédente, dans lequel le circuit de commande est configuré pour optimiser une position de déclenchement cible de l'instrument chirurgical en fonction de la force de charge résistive calculée.

11. Produit programme d'ordinateur comprenant des instructions qui, soit :
lorsqu'elles sont exécutées par le circuit de commande de l'instrument chirurgical selon la revendication 1, amènent le circuit de commande (620, 710, 760) à :

mesurer une tension de batterie activée pendant une activation du moteur pour déplacer l'actionneur ;

identifier une valeur de modulation de largeur d'impulsion (PWM) associée à l'activation de moteur ;

calculer une vitesse d'actionneur en fonction d'un changement de position capté de l'actionneur ; et

calculer la force de charge résistive en fonction de la tension de batterie activée, de la valeur PWM et de la vitesse d'actionneur ; soit

lorsqu'elles sont exécutées par le circuit de commande (620, 710, 760) de l'instrument chirurgical selon la revendication 4, amènent le circuit de commande à :

déterminer une tension de batterie de ligne de base ;

mesurer une tension de batterie activée pendant une activation du moteur ;

calculer une vitesse de l'actionneur en fonction d'un changement de position capté de l'actionneur ; et

calculer une force de charge résistive en fonction de la tension de batterie de ligne de base, de la tension de batterie activée et de la vitesse d'actionneur.

12. Support lisible par ordinateur sur lequel est stocké, ou signal transportant, le produit programme d'ordinateur selon la revendication 11.

FIG. 1

FIG. 2

FIG. 3

FIG. 4

PROCESSOR
502

INSTRUCTION
PROCESSING UNIT
506

INSTRUCTIONS

ADDRESSES

CONTROL

ARITHMETIC UNIT
508

DATA

MEMORY
504

500

FIG. 5

510

INPUT
514

COMBINATIONAL
LOGIC
512

OUTPUT
516

FIG. 6

520

INPUT
526

COMBINATIONAL
LOGIC
522

OUPUT
528

MEMORY
524

CLOCK
529

FIG. 7

FIG. 8

FIG. 9

EP 4 255 318 B1

FIG. 10

FIG. 11

2100

2102

Measure an activated battery voltage
during an activation of a motor
of the surgical instrument

2104

Identify a pulse width modulation (PWM)
value associated with the motor activation

2106

Calculate an actuator velocity based on
a sensed position change of an actuator
of the surgical instrument

2108

Identify a velocity constant based on
the actuator and a volt characterization
of the motor

2110

Determine an open loop actuator velocity
based on the activated battery voltage,
the PWM, and the velocity constant

2112

Determine a maximum output force
of the surgical instrument

2114

Calculate a resistive load force based on
the maximum output force, the open loop
actuator velocity, and the actuator velocity

FIG. 12

Force vs Velocity at Various Input Voltages

FIG. 13

2200

2202
Determine a baseline battery voltage
of the surgical instrument

2204
Measure an activated battery voltage
during an activation of a motor
of the surgical instrument

2206
Calculate a battery voltage decrease
based on a difference between the
activated battery voltage and
the baseline battery voltage

2208
Calculate a battery output power based
on the battery voltage decrease

2210
Calculate and actuator velocity based
on a sensed position change of an
actuator of the surgical instrument

2212
Determine an efficiency factor
on the surgical instrument

2114
Calculate a resistive load force based on
the efficiency factor, the battery output
power, and the actuator velocity

FIG. 14

Resistive Load Force, Battery Output Power, Battery Voltage Decrease,
and Actuator Velocity vs. Time

—— Velocity    — — —FTF (avg)    ------ Voltage drop    —— Power Output

FIG. 15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009090763 A1
- US 2019200977 A1
- US 9072535 B
- US 20140263541
- US 20170296213
- US 9345481 B
- US 20140263552
- US 62817517
- US 940632 A
- US 63682917 A
- US 72085217 A
- US 62817517 A
- US 63609617 A